# EUROPEAN PATENT APPLICATION

(11) **EP 2 876 156 A1**
(43) Date of publication of application: **27.05.2015**
(21) Application number: 13306620.9
(22) Date of filing: 26.11.2013
(51) Int. Cl.: C12N 9/02, C12P 13/04

(54) **New enzymes and method for preparing hydroxylated L-lysine or L-ornithine and analogs thereof**

(71) Applicant: COMMISSARIAT A L'ENERGIE ATOMIQUE ET AUX ENERGIES ALTERNATIVES, 75015 Paris (FR)
(72) Inventor: Zaparucha, Anne, 91440 Bures Sur Yvette (FR); De Berardinis, Véronique, 75020 Paris (FR); Saaidi, Pierre-Loïc, 91710 Vert-Le-Petit (FR); Baud, Damien, 91000 Evry (FR)
(74) Representative: Verhaeghe Bect, Elodie

(57) **Abstract**

The present invention relates to new enzymes displaying L-lysine or L-ornithine hydroxylase activity and their uses for preparing compounds of interest, in particular hydroxy-L-lysine, hydroxy-L-ornithine and hydroxylated diamino alkanes.

SEQUENCE LISTING

<110>  Commissariat à l'énergie atomique et aux énergies
       alternatives

<120>  NEW ENZYMES AND METHOD FOR PREPARING HYDROXYLATED L-LYSINE OR
       L-ORNITHINE AND ANALOGS THEREOF.

<130>  B1653EP01

<160>  16

<170>  PatentIn version 3.5

<210>  1
<211>  358
<212>  PRT
<213>  Catenulispora acidiphila

<400>  1

Met Lys Asn Leu Ser Ala Tyr Glu Val Tyr Glu Ser Pro Lys Thr Ser
1               5                   10                  15

Gly Glu Ser Arg Thr Glu Ala Val Ser Glu Ala Ala Phe Glu Ser Asp
            20                  25                  30

Pro Glu Val Ser Ala Ile Leu Val Leu Thr Ser Ser Glu Ala Ser Thr
        35                  40                  45

Leu Glu Arg Val Ala Asp Leu Val Thr Ala His Ala Leu Tyr Ala Ala
    50                  55                  60

His Asp Phe Cys Ala Gln Ala Gln Leu Ala Ala Ala Glu Leu Pro Ser
65                  70                  75                  80

Arg Val Val Ala Arg Leu Gln Glu Phe Ala Trp Gly Asp Met Asn Glu
                85                  90                  95

Gly His Leu Leu Ile Lys Gly Leu Pro Gln Val Arg Ser Leu Pro Pro
            100                 105                 110

Thr Pro Thr Ser Asn Val His Ala Val Ala Ala Thr Thr Pro Met Ser
        115                 120                 125

Arg Tyr Gln Ala Leu Ile Asn Glu Cys Val Gly Arg Met Ile Ala Tyr
    130                 135                 140

Glu Ala Glu Gly His Gly His Thr Phe Gln Asp Met Val Pro Ser Ala
145                 150                 155                 160

Met Ser Ala His Ser Gln Thr Ser Leu Gly Ser Ala Val Glu Leu Glu
                165                 170                 175

Leu His Thr Glu Gln Ala Phe Ser Pro Leu Arg Pro Asp Phe Val Ser
        180                 185                 190

Leu Ala Cys Leu Arg Gly Asp Pro Arg Ala Leu Thr Tyr Leu Phe Ser
    195                 200                 205

Ala Arg Gln Leu Val Ala Thr Leu Thr Thr Gln Glu Ile Ala Met Leu
    210                 215                 220

Arg Glu Pro Met Trp Thr Thr Thr Val Asp Glu Ser Phe Leu Ala Glu
225                 230                 235                 240

Gly Arg Thr Phe Leu Leu Gly Phe Glu Arg Gly Pro Ile Pro Ile Leu
                245                 250                 255

Ser Gly Ala Asp Asp Asp Pro Phe Ile Val Phe Asp Gln Asp Leu Met
            260                 265                 270

Arg Gly Ile Ser Ala Pro Ala Gln Glu Leu Gln Gln Thr Val Ile Arg
        275                 280                 285

Ala Tyr Tyr Ala Glu Arg Val Ser His Cys Leu Ala Pro Gly Glu Met
    290                 295                 300

Leu Leu Ile Asp Asn Arg Arg Ala Val His Gly Arg Ser Ile Phe Ala
305                 310                 315                 320

Pro Arg Phe Asp Gly Ala Asp Arg Phe Leu Ser Arg Ser Phe Ile Val
                325                 330                 335

Ala Asp Gly Ser Arg Ser Arg His Ala Arg Ser Ser Phe Gly Arg Val
            340                 345                 350

Val Ser Ala Arg Phe Ser
        355

<210>  2
<211>  370
<212>  PRT
<213>  Flavobacterium johnsoniae

<400>  2

Met Lys Ser Gln Ser Leu Ile Glu Asp Glu Ile Pro Val Lys Glu Asn
1               5                   10                  15

Tyr Ala Tyr Gln Ile Pro Thr Ser Pro Leu Ile Val Glu Val Thr Pro
            20                  25                  30

Gln Glu Arg Asn Ile Leu Ser Asn Val Gly Ala Leu Leu Glu Lys Ala
        35                  40                  45

Phe Lys Ser Tyr Glu Asn Pro Asp Tyr Ile Glu Ala Leu His Leu Tyr
    50                  55                  60

Ser Phe Gln Leu Leu Pro Glu Arg Ile Ala Arg Ile Leu Ser Arg Phe
65                  70                  75                  80

Gly Thr Asp Phe Ser Ala Asp Gln Tyr Gly Ala Ile Ile Phe Arg Gly
                85                  90                  95

Leu Leu Glu Val Asp Gln Asp His Leu Gly Pro Thr Pro Ala Asn Trp
            100                 105                 110

Gln Ser Ala Asp Tyr Ser Lys Leu Asn Lys Tyr Gly Phe Ile Cys Ser
        115                 120                 125

Leu Leu His Gly Ala Val Pro Ser Lys Pro Val Gln Tyr Tyr Ala Gln
    130                 135                 140

Arg Lys Gly Gly Gly Ile Leu His Ala Val Ile Pro Asp Glu Lys Met
145                 150                 155                 160

Ala Ala Thr Gln Thr Gly Ser Gly Ser Lys Thr Asn Leu Tyr Val His
                165                 170                 175

Thr Glu Asp Ala Phe Leu Leu His Gln Ala Asp Phe Leu Ser Phe Leu
            180                 185                 190

Tyr Leu Arg Asn Glu Glu Arg Val Pro Ser Thr Leu Tyr Ser Val Arg
        195                 200                 205

Ser His Gly Lys Val Asn Lys Ile Met Glu Lys Leu Phe Asp Pro Ile
    210                 215                 220

Tyr Gln Cys Pro Lys Asp Ala Asn Tyr Gln Glu Glu Ile Asn Asp Gly
225                 230                 235                 240

Pro Leu Ala Ser Val Leu Tyr Gly Asn Lys Lys Leu Pro Phe Ile Arg
                245                 250                 255

Phe Asp Ala Ala Glu Gln Ile Phe Asn Glu Asn Ala Gly Gln Thr Pro
            260                 265                 270

Glu Ala Leu Tyr Asn Leu Thr Glu Phe Trp Asn Glu Ala Lys Glu Leu

275                 280                 285

Ile Asn Ser Asp Tyr Ile Pro Asp Ser Gly Asp Val Ile Phe Val Asn
    290                 295                 300

Asn His Leu Cys Ala His Gly Arg Ser Ala Phe Thr Ala Gly Gln Lys
305                 310                 315                 320

Glu Glu Asn Gly Lys Leu Val Pro Cys Glu Arg Arg Gln Met Leu Arg
                325                 330                 335

Met Met Ser Lys Thr Ser Leu Ile His Ile Arg Ser Met Thr His Thr
            340                 345                 350

Asp Asp Pro Tyr Phe Val Met Glu Glu His Leu Gly Lys Val Phe Asp
        355                 360                 365

Gln Ala
    370

<210>  3
<211>  382
<212>  PRT
<213>  Chitinophaga pinensis

<400>  3

Met Arg Pro Leu Asp Val Thr Pro Thr Ile Ser Pro Gly Ala Gln Asp
1               5                   10                  15

Leu Pro Arg Thr Met His Phe Ala Ala Glu Pro Pro Leu Gln Pro Leu
            20                  25                  30

Ile Ile Asp Ile Thr Glu Glu Glu Lys Leu Glu Ile Thr Tyr Ile Gly
        35                  40                  45

Lys Lys Leu Lys Arg Lys Tyr Lys Ser Tyr Asp Asp Pro Gly Phe Ile
    50                  55                  60

Ser Met Leu His Leu Asn Ala Tyr Thr Leu Leu Pro Glu Arg Ile Ala
65                  70                  75                  80

Lys Val Leu Ser Asn Phe Gly Thr Asp Phe Ser Asp Gln Gln Tyr Gly
                85                  90                  95

Ala Val Val Leu Arg Gly Leu Ile Glu Ile Gly Gln Asp Glu Leu Gly
            100                 105                 110

Pro Thr Pro Arg Ser Trp Gln Glu Thr Asp His Glu Lys Ile Met Glu

115                 120                 125

Tyr Gly Phe Ile Ser Ser Leu Leu His Gly Ala Val Pro Ser Lys Pro
    130                 135                 140

Val Glu Tyr Phe Ala Gln Arg Lys Gly Gly Gly Leu Met His Ala Ile
145                 150                 155                 160

Ile Pro Asp Glu Asn Met Ser Phe Thr Gln Thr Gly Ser Gly Ser Arg
                165                 170                 175

Thr Asp Leu Phe Val His Thr Glu Asp Ala Phe Leu His Asn Ala Ala
            180                 185                 190

Asp Phe Leu Ser Phe Leu Phe Leu Arg Asn Glu Glu Arg Val Pro Ser
        195                 200                 205

Thr Leu Tyr Ser Ile Arg Ser His Gly Arg Pro Asp Ala Ile Leu Gln
    210                 215                 220

Glu Leu Phe Lys Pro Ile Tyr Lys Cys Pro Lys Asp Ala Asn Tyr Ala
225                 230                 235                 240

Ser Glu Glu Ala Leu Gly Asp Asp Ile Arg Thr Ser Val Leu Tyr Gly
                245                 250                 255

Ser Arg Ser Ala Pro Phe Met Arg Phe Asp Ala Ala Glu Gln Ile Tyr
            260                 265                 270

Asn Glu Asp Ala Asn Gln Asp Pro Glu Ala Leu His Asn Leu Lys Arg
        275                 280                 285

Phe Trp Glu Glu Ala Arg Lys Leu Ile Tyr Asn Asp Phe Val Pro Glu
    290                 295                 300

Ser Gly Asp Leu Ile Phe Val Asn Asn His Leu Cys Ala His Gly Arg
305                 310                 315                 320

Asn Ala Phe Leu Ala Gly Phe Arg Glu Glu Asn Gly Gln Leu Val Lys
                325                 330                 335

Cys Glu Arg Arg Leu Met Leu Arg Met Met Ser Lys Thr Ser Leu Ile
            340                 345                 350

Asn Ile Arg Glu Val Thr His Pro Glu Asn Pro Tyr Leu Ile Met Glu
        355                 360                 365

Glu His Tyr Gly Lys Val Tyr Ser Ala His Leu Ala Asn Leu
    370                 375                 380

<210>  4
<211>  343
<212>  PRT
<213>  Catenulispora acidiphila

<400>  4

Met His Arg Leu Ala Leu Thr Ala Gln Asp Asn Leu Ala Val Ala Pro
1               5                   10                  15

Met Leu Ala Asp Leu Ala Gly Arg Tyr Pro Asp Ile Glu Asp Pro Glu
            20                  25                  30

Leu Ile Arg Ser Ala Pro Val Leu Ala Ala Lys Gly Leu Pro Pro His
        35                  40                  45

Leu Leu Ala Phe Leu Asp Asp Phe Arg Leu Arg Glu Pro Ser Ala Leu
    50                  55                  60

Cys Val Ile Ser Gly Leu Asp Val Asp Gln Asp Arg Leu Gly Pro Thr
65                  70                  75                  80

Pro Glu His Trp Arg Asp Ser Gln Ile Gly Ser Arg Ser Leu Asn Leu
                85                  90                  95

Glu Ile Phe Phe Leu Leu Cys Gly Ala Ala Leu Gly Asp Val Phe Gly
            100                 105                 110

Trp Ala Thr Gln Gln Asp Gly Arg Ile Met His Asp Val Leu Pro Ile
        115                 120                 125

Lys Gly His Glu His Tyr Glu Leu Gly Ser Asn Ser Leu Gln His Leu
    130                 135                 140

Ser Trp His Thr Glu Asp Ser Phe His Pro Cys Arg Gly Asp Tyr Val
145                 150                 155                 160

Ala Leu Met Cys Leu Lys Asn Pro Tyr Glu Ala Glu Thr Met Val Cys
                165                 170                 175

Asp Ala Gly Asp Leu Asp Trp Pro Asn Leu Asp Val Asp Ala Leu Phe
            180                 185                 190

Glu Pro Val Phe Thr Gln Met Pro Asp Asn Ser His Leu Pro Gln Asn
        195                 200                 205

Thr Ala Glu Ser Thr Gly Asp Pro Thr Lys Asp Arg Leu Arg Ala Arg
    210                 215                 220

Ser Phe Glu Leu Ile Lys Ser Trp Asn Glu Asn Pro Val Arg Arg Ala
225                 230                 235                 240

Val Leu Tyr Gly Asp Arg Gln Asn Pro Tyr Met Ala Leu Asp Pro Tyr
                245                 250                 255

His Met Lys Met Asp Asp Trp Ser Glu Arg Ser Leu Glu Ala Phe Gln
            260                 265                 270

Ala Leu Cys Glu Glu Ile Glu Ala Lys Met Gln Asp Val Val Leu His
        275                 280                 285

Pro Gly Asp Ile Ala Phe Ile Asp Asn Phe Arg Ala Val His Gly Arg
    290                 295                 300

Arg Ser Phe Arg Ala Arg Tyr Asp Gly Ser Asp Arg Trp Leu Lys Arg
305                 310                 315                 320

Leu Asn Ile Thr Arg Asn Leu Arg Gly Ser Arg Ala Trp Arg Pro Ala
                325                 330                 335

Pro Asp Asp Arg Val Ile Tyr
            340

<210>  5
<211>  1077
<212>  DNA
<213>  Catenulispora acidiphila

<400>  5
atgaagaacc tgtctgcgta tgaagtgtat gaatcgccga agaccagcgg agagtcacgt      60

accgaagccg tttcggaggc cgccttcgaa tccgaccccg aagtgagcgc aatcctagtc     120

ctgacctcct ccgaagcctc gacgctggag cgcgtcgccg acctcgtgac cgcgcacgcg     180

ctgtatgccg ctcacgattt ctgtgcccag gcccagctgg ccgccgccga actgccgtcg     240

cgggtcgttg cccggttgca ggagttcgcc tggggcgaca tgaacgaggg ccaccttctg     300

ataaaaggcc tgccgcaggt ccgctccctg ccaccgacac cgacgtccaa cgtgcacgcc     360

gtcgccgcga cgacgccgat gtcgcgctac caggccctca tcaatgagtg cgtggggcgc     420

atgattgcct acgaggccga ggggcacggc cacaccttcc aggacatggt cccgagcgcc     480

atgagcgcgc actcgcagac cagtctcggc tcggcggttg agctcgaact gcacaccgag     540

caggccttca gtccgctgcg tccggacttc gtcagcctgg cctgtcttcg cggcgatcca     600

cgagccctga cttacctgtt ctcggcccgg caactggtcg cgacgctgac gacacaggaa     660

atcgccatgc tgcgcgagcc catgtggacc accaccgtcg acgagtcctt cctggcggag 720

gggcgtacgt tcctgctcgg cttcgagcgc ggtccgatcc cgatattgag cggtgccgac 780

gacgatccgt tcatcgtctt cgaccaggat ctgatgcggg ggatctcggc gcccgcgcag 840

gaactccaac agactgtgat ccgcgcctac tatgccgagc gcgtcagcca ctgcctcgca 900

cccggtgaga tgctcctcat cgacaaccgg cgcgccgtcc acggcaggtc gatcttcgcc 960

ccgcgtttcg acggcgctga ccggttcctt tccagaagct tcatcgtggc cgacggatcg 1020

cgcagtcggc acgcgcgttc ttccttcggc cgcgtcgtct ctgcgaggtt cagctga 1077

<210> 6
<211> 1113
<212> DNA
<213> Flavobacterium johnsiae

<400> 6
atgaaatcac aatcattaat tgaagatgag ataccagtaa aagaaaacta tgcttatcaa 60

attcctacaa gcccgctgat agtggaggtt acgcctcagg aaagaaacat tttgtctaat 120

gtgggcgctc tgctggaaaa ggcatttaag agctatgaaa acccagatta tatagaagcg 180

cttcatctgt attcttttca gcttcttcca gaaagaatag ccagaatttt aagccgtttt 240

ggaacagatt tctcagctga tcagtatggc gctattattt ttagaggtct tcttgaagtt 300

gatcaggatc atctgggacc aactcctgcg aattggcaga gcgctgatta ctcaaaactc 360

aataaatacg gctttatttg ttccttgctg catggtgcag ttccttcaaa accagtacaa 420

tattatgcgc agagaaaggg cgggggaatt cttcatgctg ttattccaga tgagaaaatg 480

gcagctacgc aaacaggttc gggatcaaaa acaaatttgt atgttcatac agaagatgct 540

tttcttttac atcaggctga ttttttaagt tttctatatc tgcgaaatga agaaagagtt 600

ccttctacac tttactcagt aaggtcgcat ggtaaggtga ataagataat ggaaaagctt 660

tttgatccaa tttatcaatg tcctaaagat gctaattatc aggaagaaat taatgatggt 720

ccgctggctt ctgttttata tggaaataaa aagctgcctt ttattagatt tgatgcagca 780

gagcagatat ttaatgaaaa cgccggacag actcccgaag ctctttacaa tttaactgaa 840

ttttggaatg aagctaaaga gttgattaat agtgattata tcccagattc tggtgatgtt 900

atatttgtaa ataatcattt gtgtgctcac ggaagaagtg cttttacagc agggcagaaa 960

gaggagaatg gtaagcttgt gccatgtgag agacgacaaa tgttaagaat gatgagcaaa 1020

accagtctaa ttcatataag atcaatgaca cataccgatg atccgtattt tgttatggaa 1080

gaacatttag gaaaagtttt tgatcaggct taa 1113

<210> 7
<211> 1149

<212>  DNA
<213>  Chitinophaga pinensis

<400>  7
atgagaccct tagacgtgac acccacaatt agcccaggag cccaggacct tccgcgcact      60

atgcattttg ctgctgaacc tcctttacag cctttgataa tagatattac tgaagaagaa     120

aaactggaaa ttacctatat cgggaaaaag ctaaaaagaa agtataaaag ctatgatgat     180

cccggtttta tttcaatgct gcacttaaat gcctatacgc tgctaccgga gcgtatagca     240

aaggtgctga gtaatttcgg tacagacttt tccgaccagc aatacggagc tgtcgtattg     300

cgtggactga tagaaatagg tcaggatgaa ttaggcccaa ccccacgttc ctggcaggaa     360

accgaccatg aaaagattat ggaatatggc ttcatttcct ccttattaca tggcgctgta     420

ccatccaaac ccgtcgagta tttcgcgcag cgaaaaggtg gtggcttaat gcacgcgatt     480

attcctgatg agaatatgag ctttacacaa acaggctcag gttcccgtac agatcttttt     540

gtacatacag aagatgcttt cctgcataat gcggctgatt ttctgagttt tcttttcctg     600

cggaatgaag aacgtgtgcc ttccacctta tattctatcc gctctcatgg cagaccggat     660

gcgatattac aggagctttt caagcctatc tataagtgtc cgaaggatgc gaactatgct     720

tccgaagaag ccctgggaga tgacatccgt acttctgttt tatatggtag cagatccgct     780

cccttcatgc gctttgatgc tgcggaacag atttataatg aagacgccaa tcaggatcct     840

gaagctttac ataatctgaa aagattctgg gaagaggcgc gcaaactgat atataatgac     900

ttcgttcctg agtcaggtga cctgatcttt gtgaataatc atctttgtgc ccatggccgg     960

aatgctttcc tggcaggctt cagagaggaa aatggtcagc tggtaaaatg cgaacgccgt    1020

cttatgttac gtatgatgag caaaaccagc ctgattaaca tccgtgaagt aacccacccc    1080

gaaaaccctt atctcatcat ggaagagcac tacggaaaag tatatagcgc tcacctggca    1140

aacctttaa                                                            1149

<210>  8
<211>  1032
<212>  DNA
<213>  Catenulispora acidiphila

<400>  8
atgcaccgct tggccctgac cgcgcaggac aacctggccg tggcgccgat gctcgccgat      60

ctcgccgggc gctacccgga catcgaggac ccggagctga tccgctccgc gccggtcctg     120

gcagccaaag ggctgccgcc gcacctgctg gcgttcctgg acgacttccg cctgcgcgaa     180

ccctcggcac tgtgcgtcat atcagggctc gatgtcgatc aggaccgcct gggcccgacc     240

cccgaacact ggcgcgacag ccagatcggc tcgcgctcgc tgaacctgga gatcttcttc     300

ctgctgtgcg gagcggccct cggcgacgtc ttcggctggg cgacccagca ggacggccgc     360

atcatgcacg acgtgctccc gatcaagggc cacgagcact acgagctggg ctccaactcg 420

ctgcagcacc tgtcctggca cactgaggac tccttccacc cctgccgcgg cgactacgtg 480

gcactgatgt gtctgaagaa cccttacgag gccgagacaa tggtctgcga cgccggcgac 540

ctggactggc cgaacctcga cgtggacgcg ctgttcgagc cggtgttcac gcagatgccg 600

gacaactccc acctcccgca gaacaccgcg gagtccaccg gcgacccgac gaaggaccgc 660

ctgcgcgccc gcagcttcga gctgatcaag tcctggaacg agaacccggt tcgccgcgcg 720

gtgctctacg gagaccgaca gaacccttac atggccctgg acccgtacca catgaagatg 780

gatgactggt ccgagcgctc tctggaagcg ttccaggcgc tgtgcgagga gatcgaggcg 840

aagatgcagg acgtggtcct gcaccccggc gacatcgcct tcatcgacaa cttccgcgcg 900

gtccacggcc gccgttcgtt ccgcgctcgc tacgacggca gcgaccgctg gctcaagcgg 960

ctgaacatca cccgcaatct gcgcggctcg cgggcttggc ggccggcgcc ggacgaccgg 1020

gtcatttact ga 1032

<210> 9
<211> 371
<212> PRT
<213> Niastella koreensis

<400> 9

Met Glu Thr Ile Ile Glu Ser Arg Gln Arg Ile Asn Ser Pro Gly Val
1               5                   10                  15

Leu Pro Pro Pro Leu Ser Pro Leu Ile Val Asp Val Thr Pro Lys Glu
            20                  25                  30

Arg Ala Ser Ile Ser Asn Val Ala Asn Ile Leu Leu Lys Ala Phe Gly
        35                  40                  45

His Tyr Glu His Pro Asp Phe Ile Ser Ala Leu His Leu Asn Ala Phe
    50                  55                  60

Gln Leu Leu Pro Glu Arg Ile Ala Gly Ile Leu Ser Arg Phe Gly Thr
65                  70                  75                  80

Asp Phe Ser Arg His Gln Tyr Gly Ala Leu Val Phe Arg Gly Leu Thr
                85                  90                  95

Glu Val Asp Gln Glu Ala Leu Gly Pro Thr Pro Pro Ser Trp Lys Glu
            100                 105                 110

Thr Asp Tyr Ser Lys Leu Val Lys Tyr Gly Phe Ile Cys Ser Leu Leu
        115                 120                 125

His Gly Ala Ile Pro Ser Lys Pro Val Gln Tyr Tyr Ala Gln Arg Lys
    130                 135                 140

Gly Gly Gly Leu Leu His Ala Val Ile Pro Asp Glu Lys Met Ser His
145                 150                 155                 160

Thr Gln Thr Gly Ser Gly Ser Arg Thr Asp Leu Phe Val His Thr Glu
                165                 170                 175

Asp Ala Phe Leu Phe Asn Gln Ala Asp Phe Leu Ser Phe Leu Phe Leu
            180                 185                 190

Arg Asn Glu Glu Gln Val Pro Ser Thr Leu Tyr Ser Ile Arg Ser His
        195                 200                 205

Gly Asp Thr Asn Ala Ile Met Ala Glu Leu Phe Lys Pro Ile Tyr Lys
    210                 215                 220

Cys Pro Lys Asp Ala Asn Tyr Ala Asp Asp Glu Asn Ala Gly Glu Glu
225                 230                 235                 240

Val Thr Thr Ser Ile Leu Tyr Gly Asn Arg Glu Arg Pro Phe Ile Arg
                245                 250                 255

Phe Asp Ala Ala Glu Gln Ile Tyr Asn Glu Lys Ala Gly Gln Thr Pro
            260                 265                 270

Glu Ala Met His Asn Leu Val Arg Phe Trp Asp Glu Ala Lys Gln Leu
        275                 280                 285

Ile Tyr Asn Asp Phe Val Pro Asp Ser Gly Asp Leu Ile Phe Val Asn
    290                 295                 300

Asn His Leu Cys Ala His Gly Arg Asn Ser Phe Val Ala Gly Tyr Arg
305                 310                 315                 320

Asn Glu Asn Gly Gln Leu Val Lys Cys Glu Arg Arg Leu Met Leu Arg
                325                 330                 335

Met Met Ser Lys Thr Ser Leu Ile Asn Ile Gln Ser Val Thr Gln Leu
            340                 345                 350

Asn Asp Pro Tyr Phe Ile Met Glu Glu His Tyr Gly Lys Leu Phe His
        355                 360                 365

Ser Gln Gln
    370

<210>  10
<211>  372
<212>  PRT
<213>  Flavobacterium sp.

<400>  10

Met Lys Ser Gln Ser Ile Met Ser Val Glu Arg Ser Ala Glu Thr Ser
1               5                   10                  15

Leu Thr Leu Glu Ile Pro Thr Ser Pro Leu Ile Ile Lys Ile Thr Gln
            20                  25                  30

Gln Glu Arg Asn Ile Leu Ser Asn Val Gly Asn Leu Leu Val Lys Ala
        35                  40                  45

Phe Gly Asn Tyr Glu Asn Pro Asp Tyr Ile Ala Ser Leu His Leu His
    50                  55                  60

Ala Phe Gln Leu Leu Pro Glu Arg Ile Thr Arg Ile Leu Ser Gln Phe
65                  70                  75                  80

Gly Ser Asp Phe Ser Ala Glu Gln Tyr Gly Ala Ile Val Phe Gln Gly
                85                  90                  95

Leu Ile Glu Val Asp Gln Asp Asp Leu Gly Pro Thr Pro Pro Asn Trp
            100                 105                 110

Gln Gly Ala Asp Tyr Gly Lys Leu Asn Lys Tyr Gly Phe Ile Cys Ser
        115                 120                 125

Leu Leu His Gly Ala Val Pro Ser Lys Pro Val Gln Tyr Tyr Ala Gln
    130                 135                 140

Arg Lys Gly Gly Gly Leu Leu His Ala Val Ile Pro Asp Glu Lys Met
145                 150                 155                 160

Ala Ala Thr Gln Thr Gly Ser Gly Ser Lys Thr Asp Leu Phe Val His
                165                 170                 175

Thr Glu Asp Ala Phe Leu Ser Asn Gln Ala Asp Phe Leu Ser Phe Leu
            180                 185                 190

Tyr Leu Arg Asn Glu Glu Arg Val Pro Ser Thr Leu Tyr Ser Ile Arg
        195                 200                 205

Ser His Gly Lys Met Asn Pro Val Met Lys Lys Leu Phe Glu Pro Ile
    210                 215                 220

Tyr Gln Cys Pro Lys Asp Ala Asn Tyr Asn Asp Glu Asp Val Ala Asn
225                 230                 235                 240

Ser Gly Pro Thr Ala Ser Val Leu Tyr Gly Asn Arg Glu Leu Pro Phe
                245                 250                 255

Ile Arg Phe Asp Ala Ala Glu Gln Ile Phe Asn Glu Asn Ala Gly Gln
            260                 265                 270

Thr Ser Glu Ala Leu Gly Asn Leu Met Asp Phe Trp Asp Glu Ala Lys
        275                 280                 285

Thr Leu Ile Asn Ser Asp Tyr Ile Pro Asn Ser Gly Asp Leu Ile Phe
    290                 295                 300

Val Asn Asn His Leu Cys Ala His Gly Arg Ser Ala Phe Ile Ala Gly
305                 310                 315                 320

Gln Arg Ile Glu Asn Gly Glu Ile Ile Lys Cys Glu Arg Arg Gln Met
                325                 330                 335

Leu Arg Met Met Ser Lys Thr Ser Leu Ile His Ile Arg Ser Val Thr
            340                 345                 350

Arg Thr Asp Asp Pro Tyr Phe Ile Met Glu Glu His Leu Gly Lys Ile
        355                 360                 365

Phe Asp Leu Asp
    370

<210>  11
<211>  505
<212>  PRT
<213>  Flavobacterium johnsoniae

<400>  11

Met Gln Asn Leu Leu Asp Asn Ala Pro Asp Val Phe Val Lys Ser Ala
1               5                   10                  15

Ala Asp Asp Phe Tyr Gln Asp Ile Phe His Glu Asn Ser Gly Thr Glu
            20                  25                  30

Tyr Ala Asn Ala Ile Glu Leu Val Gln Lys Arg Val Ala Gly Phe Leu
        35                  40                  45

Glu Asn Thr Lys Ile Pro Phe Ser Gly Ile Lys Pro Asp Glu Ile Arg
    50                  55                  60

Asn Lys Val Glu Ala Val Asn Leu Asp Gln Pro Val Gln Asp Tyr Glu
65                  70                  75                  80

Thr Leu Trp Asp Glu Ile Asp Glu Ile Tyr Val Asn His Ala Thr Ala
                85                  90                  95

Tyr His Leu Pro Glu Tyr Ile Ala His Leu Asn Cys Pro Val Val Ile
            100                 105                 110

Pro Ala Leu Ala Ala Asp Val Leu Ile Ser Ala Ile Asn Ser Ser Gln
        115                 120                 125

Asp Thr Tyr Asp Gln Ser Ala Gly Gly Thr Phe Ile Glu Arg Lys Leu
    130                 135                 140

Ile Ala Trp Thr Gly Glu Gln Ile Gly Tyr Asn Val Asp Cys Asp Gly
145                 150                 155                 160

Ile Phe Thr Gly Gly Gly Ser Gln Ser Asn Leu Met Gly Leu Leu Leu
                165                 170                 175

Ala Arg Asp Tyr Phe Ala Leu Lys Tyr Leu Asn Trp Asn Ile Lys Leu
            180                 185                 190

Asn Gly Cys Pro Pro Asp Ser Ser Lys Phe Arg Ile Phe Val Ser Glu
        195                 200                 205

Lys Ser His Phe Ser Asn Gln Lys Asn Ala Ser Ile Leu Gly Leu Gly
    210                 215                 220

Glu Gln Ser Ile Val Gln Val Val Thr Asp Ser Arg Tyr Arg Met Asp
225                 230                 235                 240

Ala Glu Lys Leu Lys Gln Ala Ile Leu Glu Glu Lys Glu Lys Gly Asn
                245                 250                 255

Ile Pro Ile Ala Ile Val Ala Thr Ala Gly Thr Thr Asp Phe Gly Asn
            260                 265                 270

Ile Asp Pro Leu Ser Glu Ile Ser Val Leu Ala Lys Glu His Glu Leu
        275                 280                 285

Trp Met His Val Asp Ala Ala Tyr Gly Cys Gly Leu Leu Leu Thr Asp
    290                 295                 300

Thr Tyr Lys His Leu Leu Asn Gly Ile Glu Gln Ala Asp Ser Val Thr

305                 310                 315                 320

Ile Asp Tyr His Lys Ser Phe Phe Gln Pro Ile Cys Ser Ser Ala Phe
                325                 330                 335

Met Val Arg Asn Lys Gln His Leu His Ile Ile Lys His His Ala Asp
            340                 345                 350

Tyr Leu Asn Pro Lys Glu Gln Asp Tyr Asp Glu Leu Pro Ala Gln Ile
        355                 360                 365

Asn Lys Ser Leu Val Gln Ser Thr Arg Arg Phe Asp Ala Leu Lys Leu
    370                 375                 380

Trp Cys Thr Leu Arg Tyr Met Gly Arg Thr Lys Leu Gly Gln Tyr Thr
385                 390                 395                 400

Asp Thr Ile Ile Glu Thr Thr Lys Gln Thr Ala Glu Ile Leu Glu Asn
                405                 410                 415

Asp Lys Asp Phe Glu Leu Leu Thr Asp Ser Asp Leu Ser Val Leu Val
            420                 425                 430

Phe Arg Tyr Lys Leu Asp Gly Trp Pro Gly Asp Thr Cys Lys Met Asn
        435                 440                 445

Leu His Ile Lys Lys Lys Met Phe Phe Asn Gly Glu Val Leu Val Ala
    450                 455                 460

Ser Thr Lys Val Asn Gly Ile Phe Tyr Leu Lys Phe Thr Ile Leu Asn
465                 470                 475                 480

Pro Leu Thr Thr Ile Ala His Ile Asn Asn Ile Leu Ser Ile Ile Lys
                485                 490                 495

Lys His Gly Asn Asp Tyr Ile Phe Ser
            500                 505

<210>  12
<211>  507
<212>  PRT
<213>  Chitinophaga pinensis

<400>  12

Met Ser Thr Thr Ala Phe Ala Gly Trp Glu Gln Pro Leu Gln Asp Ile
1               5                   10                  15

Val Leu Pro Val Lys Glu Thr Tyr Lys Asp Ile Phe His Gln Gly Ser

            20                  25                  30

Ala Ala Glu Tyr Glu Asn Ala Ile Asn Gly Ala Arg Glu Leu Val Thr
        35                  40                  45

Asn Phe Leu His Arg Asn Arg Gln Pro Phe Ser Gly Val Ser Ser Thr
    50                  55                  60

Ala Leu Arg Ala Ala Phe Ala Ala Val Asp Phe Asp Thr Pro Leu Pro
65                  70                  75                  80

Asp Tyr Glu Ser Leu Leu Gln Glu Val Gln Glu Leu Tyr Val Lys His
                85                  90                  95

Ala Thr Ala Phe His Leu Pro Ala Tyr Ile Ala His Leu Asn Cys Pro
            100                 105                 110

Val Val Ile Pro Ala Val Ala Ala Glu Val Leu Ile Ala Ala Ile Asn
        115                 120                 125

Ser Ser Gln Asp Thr Trp Asp Gln Ser Ala Gly Gly Thr Leu Met Glu
    130                 135                 140

Gln Gln Leu Ile Ser Trp Thr Ala Arg Glu Ile Gly Phe Gly Thr Val
145                 150                 155                 160

Ala Asp Gly Val Phe Thr Ala Gly Gly Ser Gln Ser Asn Leu Met Gly
                165                 170                 175

Leu Leu Leu Ala Arg Asp His Tyr Ala Ile Thr Asn Gly Gly His Asn
            180                 185                 190

Ile Lys Lys Gln Gly Leu Pro Gly Asp Ala Ser Arg Tyr Arg Ile Phe
        195                 200                 205

Ile Ser Glu Lys Ala His Phe Ser Asn His Lys Gly Ala Ala Leu Leu
    210                 215                 220

Gly Leu Gly Glu Gln Ala Leu Val Glu Ile Lys Thr Asp Ala Arg Phe
225                 230                 235                 240

Arg Met Asp Ala Glu Leu Leu Glu Glu Ala Ile Ala Arg Glu Ile Gln
                245                 250                 255

Gln Gly Asn Ile Pro Ile Ala Val Val Ala Thr Ala Gly Thr Thr Asp
            260                 265                 270

Phe Gly Asn Leu Asp Pro Leu Ala Asp Ile Gly Arg Ile Ala Ala Lys
        275                 280                 285

Tyr Asn Leu Trp Tyr His Ile Asp Ala Ala Tyr Gly Cys Gly Leu Leu
    290                 295                 300

Leu Thr Glu Lys Tyr Arg His Leu Leu Asn Gly Met Glu Thr Ala His
305                 310                 315                 320

Ser Val Thr Ile Asp Tyr His Lys Ser Phe Phe Gln Pro Ile Ser Ser
                325                 330                 335

Ser Ala Phe Ile Val Lys Asp Lys Gln Tyr Leu Arg Leu Leu Arg Met
            340                 345                 350

His Val Asp Tyr Leu Asn Pro Lys Asp Glu Asp Tyr Asp Asp Leu Asn
        355                 360                 365

Gln Ile Asn Lys Ser Ile Met Gln Ser Thr Arg Arg Phe Asp Ala Leu
    370                 375                 380

Lys Leu Trp Phe Thr Leu Arg Leu Met Gly Lys Gln Lys Leu Gly Ala
385                 390                 395                 400

Tyr Thr Glu Thr Ile Ile Glu Thr Ala Glu Lys Ala Ala Glu Val Ile
                405                 410                 415

Gly Leu Asp Pro Glu Leu Glu Leu Leu Ser Tyr Ser Asp Ile Gly Val
            420                 425                 430

Ile Leu Phe Arg Tyr Ala Pro Glu Gln Tyr Asn Pro Ala Asp Leu Asp
        435                 440                 445

Ala Leu Asn Gln Tyr Ile Lys Lys Thr Met Phe Asn Thr Gly Leu Val
    450                 455                 460

Leu Val Ala Ser Thr Arg Val Asn Gly Asn Phe Tyr Leu Lys Phe Thr
465                 470                 475                 480

Met Leu Asn Pro Leu Thr Thr Leu Asp Asp Val His Phe Ile Leu Asn
                485                 490                 495

Thr Ile Lys Glu Thr Gly Ala Ala Trp Arg Gln
            500                 505

<210>  13
<211>  1116
<212>  DNA

<213>  Niastella koreensis

<400>  13
atggaaacta tcattgaatc cagacaacgc attaacagtc ccggcgtatt accgccacct      60

ttgagtccat tgatcgttga cgttacaccg aaggagcgtg cctccatttc aaacgtggcc     120

aatatcttat taaaagcctt tggccattat gaacatcctg atttcatctc cgctttgcac     180

ctgaatgctt ttcagttatt accggaacgt attgcgggga tactgagccg ttttggtacc     240

gacttctcgc gccaccaata cggcgcgttg gtgttcagag gccttacaga agtagatcag     300

gaggcgcttg gccctacccc gccctcgtgg aaagaaaccg attacagcaa gcttgttaaa     360

tatggattta tttgctcgct gctgcatggc gccattccat caaaaccagt acaatattat     420

gcgcagcgaa aaggcggtgg tttactgcat gccgttattc ccgatgaaaa aatgagtcat     480

acgcaaaccg gctccggctc gcgcaccgat ctttttgtgc ataccgaaga tgcgttctta     540

tttaaccagg ccgattttct cagcttcctg ttcctgcgga atgaagaaca ggtgccatct     600

acgttatatt cgatccggtc gcatggcgat accaacgcca tcatggcgga gctgttcaaa     660

cccatttata agtgtccgaa ggatgcgaat tatgccgacg atgaaaatgc cggcgaggaa     720

gtgaccactt ctatcttata cggtaaccgc gaacggccct ttatccgctt cgatgccgcg     780

gaacagatct acaacgaaaa ggccggacaa acgccggaag ccatgcacaa cctggtgcgt     840

ttttgggacg aagccaaaca acttatctac aatgatttcg tgcccgattc gggcgatctc     900

atttttgtaa acaaccattt gtgcgcgcat ggccggaatt catttgtggc cggttatcgt     960

aatgaaaacg gtcagctggt aaaatgtgaa cgccggttga tgttacgcat gatgagcaag    1020

accagcctca tcaatattca gtcggtgacc cagttaaacg acccgtattt cattatggaa    1080

gaacactacg gcaaattgtt tcattcacaa caataa                              1116

<210>  14
<211>  1119
<212>  DNA
<213>  Flavobacterium sp.

<400>  14
atgaaatcac aatctattat gtcagtcgaa agaagtgctg agacaagttt aactttagaa      60

attcctacaa gccctttaat tattaaaata acacagcagg agagaaacat tttatcaaac     120

gttggtaatt tgttggtaaa agcatttggc aattatgaaa acccggatta tatagcctct     180

cttcatcttc atgcttttca attgcttcct gagagaataa ccagaatctt aagtcagttt     240

ggttcagatt tttctgctga acaatatggt gctatagtct ttcagggact tatagaagta     300

gatcaggatg atttggggcc aacgccaccc aactggcagg gagctgatta tggaaaatta     360

aacaaatatg gttttatctg ttcgcttctt catggtgcag tgccttctaa gccggttcaa     420

tattatgccc agagaaaggg aggaggtttg ttgcatgctg ttattcctga tgagaaaatg     480

gcagccacac aaacaggttc aggttccaaa actgatttat ttgtacatac agaagatgcc 540

tttttatcca atcaggcaga ttttttaagt tttctatatc tgagaaatga agagagagta 600

ccgtctacat tatattcgat tcgttctcac ggaaaaatga atcctgtgat gaaaaagtta 660

ttcgaaccca tttatcaatg cccgaaagat gccaattata atgatgaaga tgtagcaaat 720

tcggggccta cagcctctgt attatatgga aacagagaac ttccttttat tcgttttgat 780

gctgccgaac aaatatttaa tgaaaatgca ggacaaactt ctgaggcact tggcaactta 840

atggattttt gggatgaagc caaaactttg attaatagtg attatattcc aaattcagga 900

gatcttattt ttgtgaataa tcacttgtgt gcacatggaa gaagtgcttt tattgccgga 960

cagagaatag aaaatggtga aattataaaa tgtgaacgcc gtcaaatgct aaggatgatg 1020

agtaagacaa gtttaattca tatcagatca gtaacccgta cagatgaccc ttattttatt 1080

atggaagagc atttagggaa aatttttgac ctggattag 1119

<210> 15
<211> 1518
<212> DNA
<213> Flavobacterium johnsoniae

<400> 15
atgcaaaacc tattagacaa tgccccggat gtctttgtta aaagcgccgc tgacgatttt 60

tatcaggata tttttcatga aaattccgga acagaatatg ccaatgccat agaattagtt 120

caaaaacgtg tagcaggttt tcttgaaaat actaaaatcc cgttcagcgg tattaaacct 180

gacgaaatac gaaataaagt agaagctgta aaccttgatc agccagttca ggattatgaa 240

accctttggg acgagatcga tgaaatttat gtaaatcatg ccactgctta ccatttacca 300

gaatatatag cccacttaaa ctgccctgtt gtaattcctg ctttagcagc cgatgtattg 360

attagcgcta ttaactcatc gcaagacacg tatgaccaaa gtgcaggtgg aacattcatt 420

gaaagaaaat tgattgcctg gactggcgaa caaattggat ataatgtaga ttgtgatggt 480

atttttactg gcggcggatc acaaagtaac cttatgggac tgctattggc cagagattat 540

tttgctttaa aatacctcaa ctggaacatc aaattaaatg gctgtccgcc ggattcaagt 600

aaattcagaa tttttgtttc agagaaatct catttcagca atcaaaaaaa tgcttctatt 660

ctaggtcttg gcgagcagtc tattgttcag gttgtaacag acagccgtta cagaatggac 720

gctgaaaaat taaagcaggc cattcttgaa gaaaaagaaa aaggcaacat tcctattgct 780

attgtggcta cagcaggaac aacagatttt gggaacatcg atccattatc tgaaatttca 840

gttttggcaa aagaacatga attatggatg catgttgacg ccgcttatgg ctgcgggtta 900

ttgctgacag atacatacaa acatttgtta aacggaattg aacaagctga ttctgttaca 960

attgattatc ataaatcttt ctttcagcct atttgcagca gtgcttttat ggtgagaaac 1020

aaacagcatc ttcatatcat aaaacatcat gctgattatt taaatccaaa agaacaggat 1080

tacgatgaac ttccggcaca aataaacaaa tctctggtac aaagtacacg ccgttttgat 1140

gctttaaaac tatggtgtac actgcgttat atgggaagaa ctaaacttgg acagtataca 1200

gataccatta ttgaaacgac caaacaaaca gctgaaatac tggaaaacga caaagacttt 1260

gaactcttaa cagattctga tttaagtgtt ttggttttta gatacaaact agacggctgg 1320

cctggagaca cttgtaaaat gaacttacac atcaaaaaga aaatgttttt taacggtgaa 1380

gtcttagtgg caagcacaaa agtaaacggc attttttacc tcaagtttac cattttgaat 1440

ccattaacaa caatcgcaca catcaataat atcctttcaa ttataaaaaa acacggaaat 1500

gactacatct tctcctaa 1518

<210> 16
<211> 1524
<212> DNA
<213> Chitinophaga pinensis

<400> 16
atgagtacta ccgcatttgc aggctgggaa cagccgctgc aggacattgt attgcctgta 60

aaagagacct ataaagatat ttttcatcaa ggatcggctg ctgaatatga aaacgccatc 120

aatggcgcac gcgaactggt cactaatttt ctgcaccgga atcgccagcc tttcagcggc 180

gtctcttcta ctgcactgag agcagcattt gcagcagtag attttgatac cccattacct 240

gattacgaaa gtctcctgca ggaagtacag gaactatatg taaaacatgc cactgccttt 300

catctgccag catacatcgc acatctgaat tgcccggtgg tgataccggc agttgcagct 360

gaagtactga ttgctgccat caattcttct caggatacct gggaccagag tgcaggcggt 420

accctgatgg aacaacagct gattagctgg acggcacgtg aaattggctt cggtacagtt 480

gctgatggtg tatttacagc gggtggttca cagagtaacc tgatgggtct attactagcc 540

agagatcact atgcgattac aaacggcggt cataatatta aaaagcaggg cttacctggg 600

gatgcttccc gttatcgcat tttcatttca gaaaaagcac atttcagcaa ccataaaggc 660

gctgcattgt taggattggg ggagcaggca ttagtagaaa taaaaacaga tgcacgcttc 720

cgtatggatg cggaactgct ggaagaagcg attgccaggg agatacaaca agggaatatt 780

cctattgcag tcgtggctac cgcaggcacc actgatttcg gtaacctgga tccgctggct 840

gatataggcc gtatcgcggc taaatacaac ctgtggtatc atatagatgc tgcctatgga 900

tgtgggctgc tgctgacgga gaaataccgt catttactga atggtatgga aacagcacat 960

tctgttacga tcgactatca caaatcattc tttcagccta tcagcagcag tgcctttatc 1020

gtgaaggata aacaatacct gcggctgtta cgtatgcatg tcgactatct caatccaaag 1080

gatgaagatt acgacgatct gaaccagatc aataagtcca tcatgcagag tacccgtcgc 1140

tttgatgcat tgaagttgtg gttcaccttg agactgatgg gtaaacagaa actcggtgcg 1200

tatacggaaa ccatcatcga aacagcggaa aaagctgcgg aagtcatcgg actggatcct 1260

gaactggaac tgctgagtta ttcggatatc ggtgtcatcc tgttcagata tgcaccggaa 1320

cagtataatc ctgccgatct ggatgccctg aaccaataca tcaaaaagac gatgttcaac 1380

accggattgg tattagtggc aagtacacgt gtcaacggta acttctatct gaagtttact 1440

atgctgaatc cgttgaccac cctggacgat gtacatttca ttcttaacac cattaaagag 1500

actggtgctg catggcgcca ataa 1524

## Description

### FIELD OF THE INVENTION

The present invention relates to new enzymes and their use for preparing compounds of interest.

### BACKGROUND OF THE INVENTION

Chiral compounds are key intermediates for the synthesis of valuable compounds, especially in the pharmaceutical field wherein chirality plays a crucial role in biological activity.

Among them, hydroxylated L-lysine and L-ornithine are of a high interest due to their two chiral centers and their functional groups which can be easily derivatised. Hydroxylated L-lysine and L-ornithine may thus serve as building blocks for the synthesis of potential drugs such as antibiotics and anticancer agents or as chiral ligands in asymmetric synthesis. For instance, (3R)-hydroxy-L-lysine is a key intermediate for the synthesis of (-)-balanol, a potent inhibitor of human protein kinase C. On the other hand, the diasteroisomers (2S,3R) and (2S,3S) of hydroxy-ornithine may be useful building blocks for the preparation of analogs of avicin, an anticancer drug, and those of the antibiotic streptothricin respectively.

The state of the art describes several chemical methods for preparing hydroxylated L-lysine or L-ornithine in optically pure forms.

For instance, Masse et al. (Organic Letters, 2000, 17, 2571-2573) describe the synthesis of (2S,3R)-hydroxylysine ((3R)-hydroxy-L-lysine) from 4-chloro-1-butanol. The synthesis scheme comprises 6 steps. Briefly, the Swern oxidation of 4-chloro-1-butanol provided 4-chlorobutanal which was subjected to a Horner-Emmons olefination so as to obtain (para-bromophenyl)-6-chloro-2-(E)-hexenoate. The resulting compound was subjected to an asymmetric aminohydroxylation to create C3-C4 stereogenic centers whereby (2S,3R)-(para-bromophenyl)-2-benzylcarbamate-3-hydroxy-6-chlorohexanoate is obtained with a good regioselectivity and enantioselectivity. Then, the primary chloride of the resulting α-amino-β-hydroxy ester is replaced by an azide group (NaN₃/DMF). (3R)-hydroxy-L-lysine is finally obtained by a step of hydrogenation (to reduce azide moiety into amino group and remove Cbz group) followed by saponification in acidic condition.

Wityak et al. (J. Org. Chem, 1987, 52, 2179-2183) disclose a multistep method for preparing (3R)-hydroxy-L-ornithine and (3S)-hydroxy-L-ornithine from protected (2S)-vinylglycine. Briefly, this method comprises the generation of a diastereoisomeric mixture of isoxazolidines from the protected (2S)-vinylglycine, and the separation of isoxazolidine diasteoisomers by flash chromatography. Finally, (3R)-hydroxy-L-ornithine and (3S)-hydroxy-L-ornithine are obtained from the appropriate isoxazolidine diastereoisomer by metal reduction followed by hydrogenolysis.

It is thus apparent that the chemical synthesis of hydroxylated L-lysine or L-ornithine, especially in pure diastereoisomeric form, is still tricky and time-consuming. Moreover, these chemical synthesis are difficult to implement at industrial scale because of economic and environmental concerns.

Biocatalysis is more and more used in chemical industry due to high regio- and stereoselectivity, mild conditions and reduced waste. Unfortunately, there is a very few examples of enzymes able to hydroxylate L-lysine or L-ornithine. Certain flavoprotein monooxygenases such as PvdA from *Pseudomonas aeroginosa,* SidA from *Aspergillus fumigatus* or LucD from *Escherichia Coli,* catalyze the hydroxylation of the amino group of the side-chain of ornithine or lysine and thus exhibit N-hydroxylase activity (Olucha and Lamb, Bioorg. Chem, 2011, 39(5-6):171-177). However, these enzymes are unable to introduce a hydroxyl group on a carbon of the side chain of L-lysine or L-ornithine.

On the other hand, lysyl hydroxylases (protocollagen-lysine 2-oxoglutarate 5-dioxygenase) are the enzymes responsible of the hydroxylation of lysyl residues found in collagens and proteins having collagen-like domains. However, the hydroxylation capability of these enzymes is quite restricted. Lysyl hydroxylases introduce the hydroxyl group on position δ, namely on carbon C-5 of L-lysine residue only. Moreover, lysyl hydroxylases are totally inefficient to hydroxylate lysine or ornithine as free amino acids *in vitro* and *in vivo.* Indeed, the hydroxylation reaction mediated by lysyl hydroxylases occurs during collagen biosynthesis in the endoplasmic reticulum as a co- and post-translational event, and not on free amino acids. The *in vitro* minimum sequence requirement for lysyl hydroxylase is a tripeptide of formula X-Lys-Z wherein X is an amino acid and Z is Gly, Ser or Ala.

The stereoselective hydroxylation of free L-lysine or L-ornithine on carbon position of their side-chains is thus accessible by complex multistep chemical synthesis only.

Therefore, there is a need of new methods for producing hydroxylated L-lysine and L-ornithine and derivatives thereof, especially with controlled stereochemistry and regioselectivity

### SUMMARY OF THE INVENTION

In a first aspect, the invention relates to a method for hydroxylating a compound of formula (I), or a salt thereof: 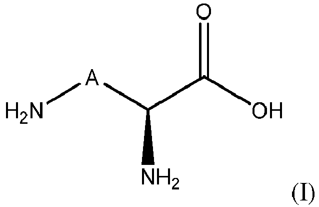 wherein A is -(CH₂)ₙ-X-(CH₂)ₚ- with X is CH₂, or CH(OH), n is an integer from 0 to 4, preferably from 1 to 4, and p is an integer from 0 to 4 with proviso that 0≤ n+p ≤ 4 wherein said method comprises
- contacting the compound of formula (I) or a salt thereof with an enzyme having a L-lysine or L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4; and
- optionally recovering the hydroxylated compound.

Preferably, the hydroxyl group is introduced on a carbon position of the moiety A of the compound of formula (I).

In some embodiments, the method of the invention is characterized by the following features:
- the compound of formula (I) is such that n is an integer from 1 to 3 and p is an integer from 0 to 2 with proviso that n + p =3, and
- the enzyme has a L-lysine hydroxylase activity and comprises an amino acid sequence having at least 70% of sequence identity with an amino acid sequence selected from the group consisting of SEQ ID NO:1, SEQ ID NO:2, SEQ ID NO:3, SEQ ID NO:9, and SEQ ID NO:10.

In some other embodiments, the method of the invention is for preparing (3S)-hydroxy-L-lysine and comprises contacting L-lysine with an enzyme capable of producing (3S)-hydroxy-L-lysine from L-lysine and comprising an amino acid sequence having at least 70% of sequence identity with SEQ ID NO: 1.

In another embodiment, the method of the invention is for preparing (4R)-hydroxy-L-lysine and comprises contacting L-lysine with an enzyme capable of producing (4R)-hydroxy-L-lysine from L-lysine and comprising an amino acid sequence having at least 70% of sequence identity with an amino acid sequence selected from the group consisting of SEQ ID NO:2, SEQ ID NO:3, SEQ ID NO:9 and SEQ ID NO:10.

In an additional embodiment, the method of the invention is for preparing (3S)-hydroxy-L-ornithine and comprises contacting L-ornithine with an enzyme capable of producing (3S)-hydroxy-L-ornithine from L-ornithine and comprising an amino acid sequence having at least 70% of sequence identity with SEQ ID NO:4.

In preferred embodiments, the enzyme comprises an amino acid sequence having at least 90%, preferably at least 95%, more preferably at least 99% of sequence identity with an amino acid sequence selected from the group consisting of SEQ ID NO:1, SEQ ID NO:2, SEQ ID NO:3, SEQ ID NO:9, SEQ ID NO:10, and SEQ ID NO:4.

In some embodiments of the method of the invention, the enzyme and the compound of formula (I) are contacted in the presence of a cofactor and a co-substrate. The co-substrate may be an α-keto acid or a salt thereof, preferably in α-ketoglutaric acid or salt thereof. The cofactor may be a source of Fe²⁺.

The enzyme may be present in a free form or may be immobilized on a support. In an alternate embodiment, the enzyme may be expressed *in situ,* namely in the reaction medium, by a wild-type cell or by a host cell capable of expressing said enzyme. The method of the invention may further comprise the purification of hydroxylated compound.

A further object of the invention is a recombinant nucleic acid construct or vector
- comprising an nucleic acid sequence encoding an enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4, and
- optionally, one or more control sequences which is/are operably linked to said nucleic acid sequence and that is/are capable to direct or control the production of the enzyme in a host cell.

The invention further relates to a recombinant host cell comprising the nucleic acid construct or vector as defined above. In an additional aspect, the invention relates to a method for producing an enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4, said method comprising:
- cultivating a cell, which in its wild-type form is capable of producing the enzyme, or a recombinant host cell of the invention, in conditions conducive for production of the enzyme, and
- recovering and/or purifying the enzyme.

The invention further relates to a solid support on which is immobilized an enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4.

A further object of the invention is a composition comprising an isolated enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4, and an excipient.

It is also provided a kit for hydroxylating a compound of formula (I) as defined above comprising: an isolated or recombinant enzyme having a L-lysine or L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4, a solid support, a composition, or a recombinant host cell as described above.

In a further aspect, the invention relates to the use of an enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4, a solid support, a kit, a composition or a recombinant host cell as defined above for hydroxylating a compound of formula (I) or a salt thereof as defined above.

At last, the invention further relates to a method for producing a compound of interest comprising:
- producing a hydroxylated compound from a compound of formula (I) or a salt thereof by the method according to the present invention and
- using the resulting hydroxylated compound or a salt thereof for preparing the compound of interest.

In a particular aspect, the invention relates to a method for preparing a hydroxylated diamino alkane, said method comprising:
(i) providing a compound of formula (I): 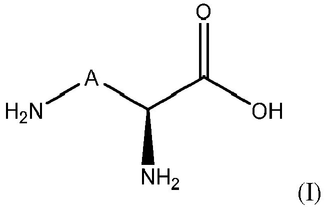 wherein A is -(CH₂)ₙ-X-(CH₂)ₚ- with X is CH₂, or CH(OH) and n is an integer from 0 to 4, preferably from 1 to 4, and p is an integer from 0 to 4 with proviso that 0≤ n+p ≤ 4, preferably n+p = 2 or 3,
(ii) hydroxylating compound of formula (I) by contacting said compound with an enzyme having a L-lysine or L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10 and SEQ ID NO:4,
(iii) decarboxylating the compound obtained in step (ii), preferably by using a decarboxylase, and
(iv) optionally recovering the resulting hydroxylated diamino alkane.

Preferred hydroxylated diamino alkanes are among others (2S)-1,5-diamino-2-pentanol, 1,5-diamino-3-pentanol, (2S)-1,4-diamino-2-butanol, 1,5-diamino-pentane-2,3-diol, in particular (2R,3R)-1,5-diamino-pentane-2,3-diol and salts thereof.

### FIGURES

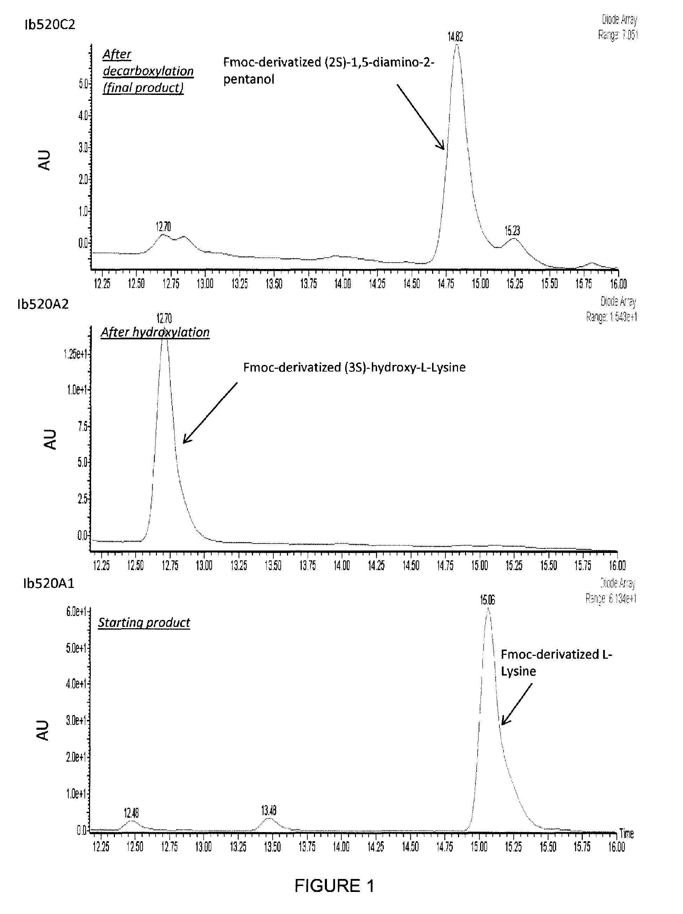

Figure 1 shows the monitoring of the enzymatic conversion of L-lysine into (2S)-1,5-diamino-2-pentanol by HPLC according to protocol A (Fmoc derivatization). From bottom to top: HPLC chromatogram obtained for the reaction medium before hydroxylation of L-lysine with ENZ1 (starting product), HPLC chromatogram of the reaction medium after hydroxylation and HPLC chromatogram obtained for the reaction medium after decarboxylation (final product).

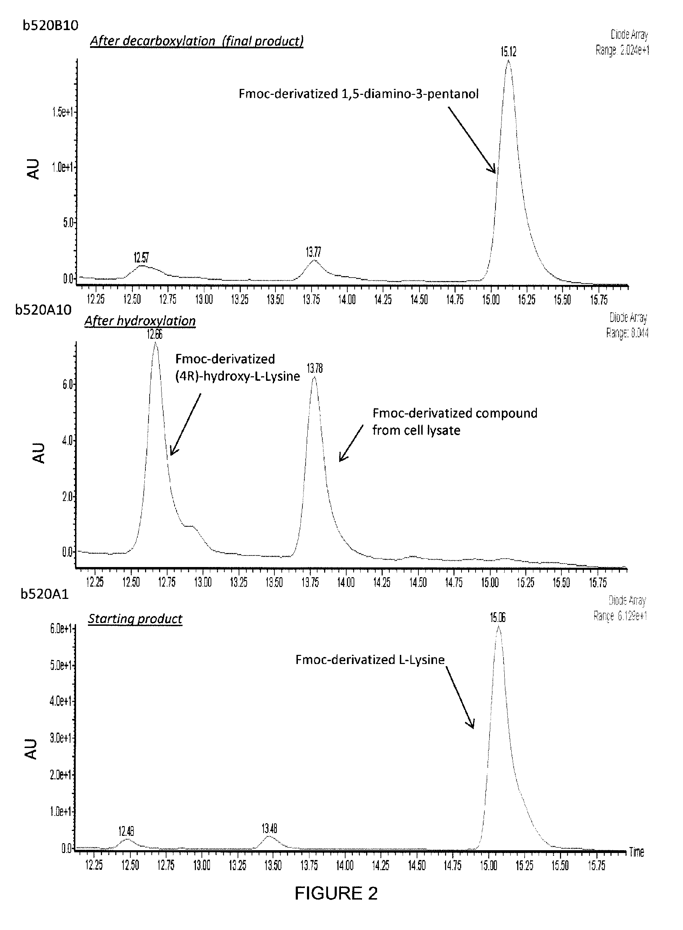

Figure 2 shows the monitoring of the enzymatic conversion of L-lysine into 1,5-diamino-3-pentanol by HPLC according to protocol A (Fmoc derivatization). From bottom to top: chromatogram obtained for the reaction medium before hydroxylation (starting product), chromatogram of the reaction medium after hydroxylation of L-lysine with ENZ2 and chromatogram obtained for the reaction medium after decarboxylation (final product).

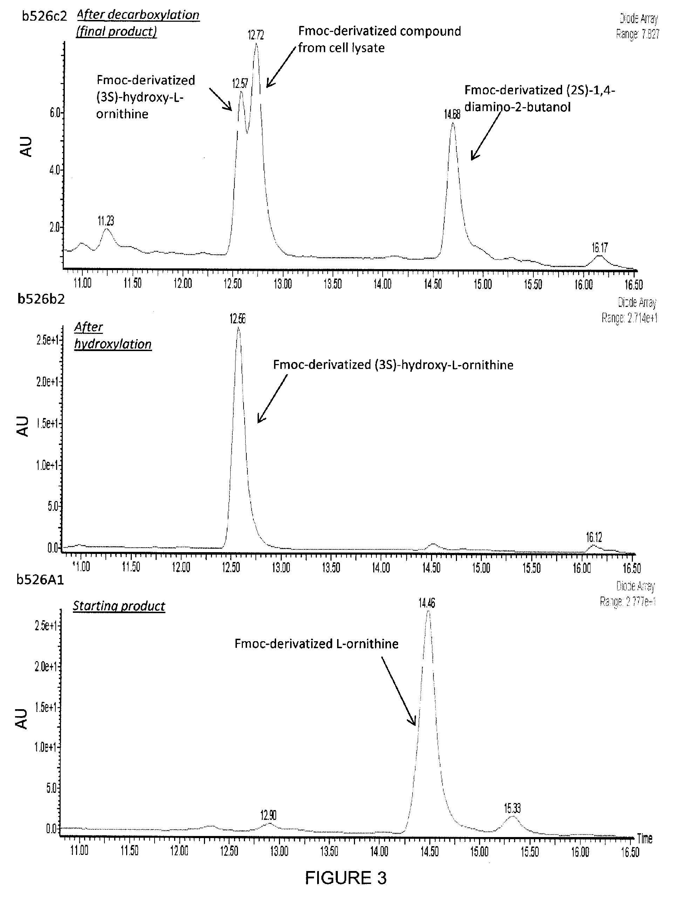

Figure 3 shows the monitoring of the enzymatic conversion of L-ornithine into (2S)-1,4-diamino-2-butanol by HPLC according to protocol A (Fmoc derivatization). From bottom to top: chromatogram obtained for the reaction medium before hydroxylation (starting product), chromatogram of the reaction medium after hydroxylation of L-ornithine with ENZ4 and chromatogram obtained for the reaction medium after decarboxylation (final product).

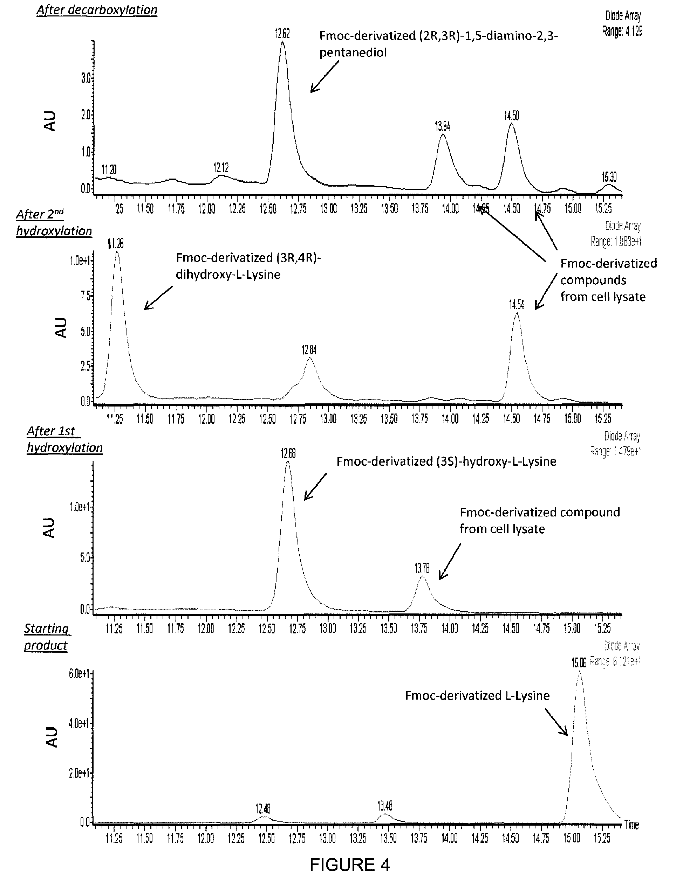

Figure 4 shows the monitoring of the enzymatic conversion of L-lysine into (2R,3R)-1,5-diamino-2,3-pentanediol by HPLC according to protocol A (Fmoc derivatization). From bottom to top: chromatogram obtained for the starting reaction medium, chromatogram obtained after hydroxylation with ENZ1, chromatogram obtained for the reaction medium after the 2^{nd} hydroxylation using ENZ2 and chromatogram obtained for the reaction medium after decarboxylation (final product).
   For all chromatograms by HPLC: X-coordinate: time in minute, Y-coordinate: UV absorption.

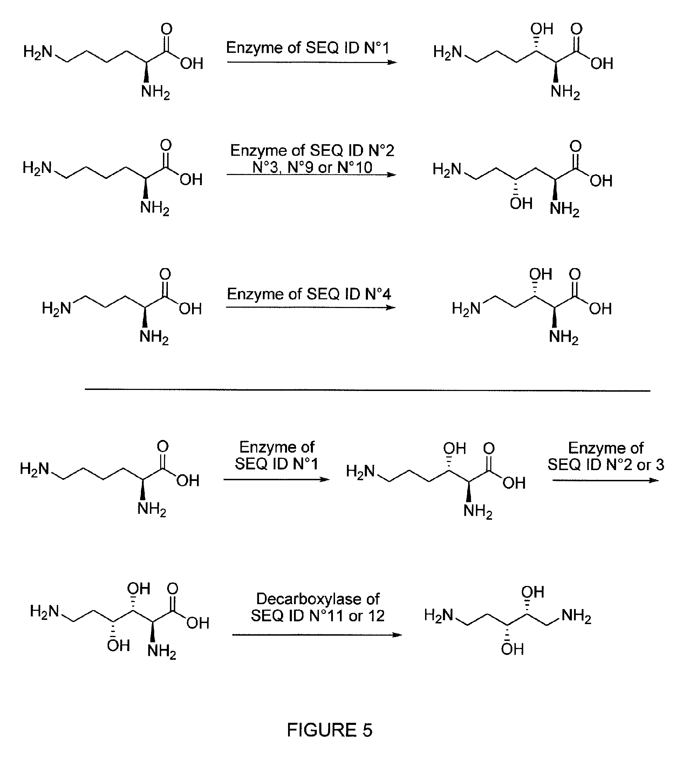

Figure 5 shows some synthesis schemas according to the invention.

### DETAILED DESCRIPTION

The inventors surprisingly identified enzymes which are able to hydroxylate free L-ornithine or free L-lysine on carbon positions of the side-chains. To the inventors' knowledge, it was the first time that such an enzymatic activity was disclosed. Noteworthy enough, the enzymes of the invention are able to hydroxylate free L-ornithine or free L-lysine with controlled regioselectivity and stereoselectivity.

The inventors further showed that these enzymes may be able to hydroxylate specific derivatives and analogs of L-ornithine and L-lysine, in particular hydroxy-L-Lysine.

In a more general aspect, the enzymes of the invention can be used to hydroxylate compound of formula (I): 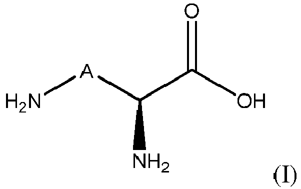 wherein A is -(CH₂)ₙ-X-(CH₂)ₚ- with X is CH₂, or CH(OH), n is an integer from 0 to 4, preferably from 1 to 4, and p is an integer from 0 to 4 with proviso that 0≤ n+p ≤ 4.

The enzymes of the invention belong to the family of α-ketoglutarate-dependent dioxygenase and thus may require a source of Fe²⁺ and a source of α-ketoglutarate or analogs thereof to catalyze *in vitro* the hydroxylation of compounds of formula (I).

### ■ Definitions

*Free amino acid:* As used herein, a free amino acid such as a free L-lysine or a free L-ornithine refers to the amino acid *per se,* in its free form. In other words, it refers to an amino acid which is not covalently linked to another chemical moiety such as another amino acid.

*Hydroxylase activity:* As used herein, an enzyme has "a hydroxylase activity" means that said enzyme is able to hydroxylate, namely to introduce a group -OH, on a carbon position of a substrate of interest. In the context of the present invention, "a hydroxylase activity" does not encompass the capability of an enzyme for hydroxylating an amino group. Such an activity is called herein *"N-hydroxylase activity".*

*L-lysine hydroxylase activity:* In the context of the invention, an enzyme displays L-lysine hydroxylase activity if said enzyme is able to catalyze the introduction of a hydroxyl group on the side-chain of a free L-lysine, namely at carbon C-2, C-3, C-4, C-5, C-6, preferably at carbon C-3, C-4 or C-5 as shown in the below scheme.

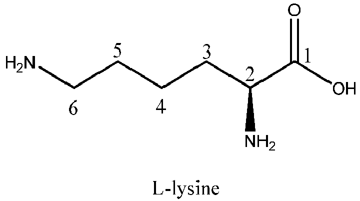

*L-ornithine hydroxylase activity:* In the context of the invention, an enzyme displays L-ornithine hydroxylase activity if said enzyme is able to catalyze the introduction of a hydroxyl group on the side-chain of a free L-ornithine, namely at carbon C-2, C-3, C-4 and C-5, preferably at carbon C-3, or C-4 as shown in the below scheme.

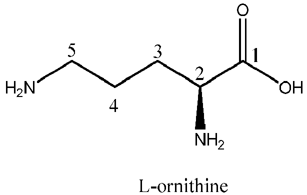

*α-ketoglutarate-dependent dioxygenase:* "α-ketoglutarate-dependent dioxygenase" refers to a superfamily of enzymes able to catalyze an oxidative reaction on a given substrate from molecular O₂. These enzymes generally require Fe²⁺ as cofactor and α-ketoglutarate as cosubstrate. The presence of ascorbate as an additional cofactor may promote enzymatic activity *in vitro.* More precisely, an α-ketoglutarate-dependent dioxygenase contains a non-haemic Fe²⁺ in its catalytic center and catalyzes the oxidation of a given substrate from molecular O₂ while leading to the oxidative decarboxylation of α-ketoglutarate into succinate. α-ketoglutarate-dependent dioxygenases may mediate a variety of oxidative reactions and are involved in various biosynthesis. Each α-ketoglutarate-dependent dioxygenase displays specific properties, especially in terms of substrate selectivity and chemical reaction. For review about α-ketoglutarate-dependent dioxygenases, see Bugg, Tetrahedon, 2003, 59, 7075-7101. As fully explained below, an enzyme of the invention may belong to α-ketoglutarate-dependent dioxygenase family and is characterized by its L-ornithine and/or L-lysine hydroxylase activity.

*Assessment of hydroxylase activity:* As explained further below, the enzyme of the invention is preferably a α-ketoglutarate-dependent dioxygenase which is able to catalyze the *in vitro* hydroxylation of an appropriate substrate, preferably L-lysine and/or L-ornithine. The reaction preferably takes place in the presence of α-keto-acid or salt thereof, preferably α-ketoglutarate (also called 2-oxoglutarate) as a co-substrate and Fe²⁺ as co-factor. The ability of the enzyme to catalyze the hydroxylation of a substrate, preferably L-lysine and/or L-ornithine, can be assessed *in vitro* by contacting the enzyme with the substrate in conditions conducive for the enzyme activity and detecting the formation of the hydroxylated substrate. *In vitro* conditions for assessing the hydroxylase activity of an enzyme according to the invention may comprise contacting the enzyme and the substrate in the presence of α-ketoglutaric acid, or a salt thereof, and a source of Fe²⁺. The presence of ascorbate may be further required. Typically, the enzymatic reaction can be implemented in a buffer at a pH ranging from 6.70 to 9.60 and at a temperature ranging from 22°C to 42°C.

An appropriate method is disclosed in details, further below, in the experimental section. For instance, the assessment of hydroxylase activity can be implemented in the following conditions:
- buffer (e.g. HEPES) at pH 7.5, at 22-42°C
- co-factor: 1 mM of Mohr's salt
- optionally up to 40 mM of sodium ascorbate
- co-substrate: 1 mM to 15 mM of α-ketoglutaric acid, and
- substrate to hydroxylate: 5 mM to 10 mM.

The amounts of each reagent depend on the amount of the active enzyme which is introduced in the medium.

*Lysine or ornithine analogs:* lysine or ornithine analogs refer to any compound which derives from lysine or ornithine, preferably from L-lysine or L-ornithine and which may be hydroxylated by an enzyme according to the invention. Preferably, analogs of L-lysine and L-ornithine encompass compounds of formula (I): 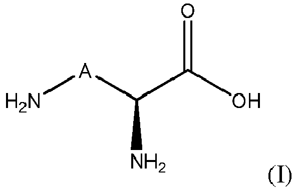 wherein A is -(CH₂)ₙ-X-(CH₂)ₚ- with X is CH₂, or CH(OH), n and p are integers from 0 to 4 with proviso that 0≤ (n+p) ≤ 4.

Preferably, n is an integer from 1 to 3, and p is an integer from 0 to 2 with proviso that (n+p) is 2 or 3.

*Hydroxylated product:* refers to the product(s) resulting from the hydroxylation of the substrate (compound of formula (I), e.g. L-lysine or L-ornithine). It does not encompass the products resulting from the oxidation of the co-substrate (e.g. succinate when α-ketoglutarate is used as cosubstrate).

*Hydroxylated diamino alkane (or a diaminoalkanol):* refers to an alkane, preferably a C₄-C₅ alkane, comprising at least two amino groups, and at least one hydroxyl group. The amino groups are preferably primary and the hydroxyl group is preferably a secondary alcohol group.

*Decarboxylase activity:* In the context of the invention, an enzyme displays a decarboxylase activity if said enzyme is able to catalyze the removal of a COOH group from an organic compound, in particular from an amino acid. Preferred decarboxylases are pyridoxal phosphate (PLP)-dependent carboxylases, in particular amino acid PLP-dependent decarboxylases such as those able to remove a COOH group from L-lysine and/or L-ornithine. For review concerning PLP-dependent decarboxylases, please refer to Jordan and Patel, ACS Catal, 2013,3,1601-17, the disclosure of which being incorporated by reference. *Coding sequence:* The term "coding sequence" or "coding nucleic acid" means a polynucleotide which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

*Control sequences:* The term "control sequences" means nucleic acid sequences necessary for expression of a polynucleotide encoding an enzyme of the present invention. Control sequences may be native (i.e., from the same gene) or heterologous (i.e., from a different gene and/or a different species) to the polynucleotide encoding the enzyme. Preferably, control sequences are heterologous. Well-known control sequences and currently used by the person skilled in the art will be preferred. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding the enzyme. The functional combination of control sequences and coding sequences can be referred as *expression cassette.*

*Expression:* The term "expression" includes any step involved in the production of a polypeptide including, but not limited to, transcription, post-transcriptional modification, translation, post-translational modification, and secretion.

*Expression vector:* The term "expression vector" means a linear or circular DNA molecule that comprises a polynucleotide encoding the enzyme of the invention and is operably linked to control sequences that provide for its expression. Then the expression vector comprises an expression cassette suitable for expressing the enzyme of the invention.

*Isolated:* The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance).

*Recombinant:* Recombinant refers to a nucleic acid construct, a vector and a protein produced by genetic engineering.

*Heterologous:* in the context of a host cell, a vector or a nucleic acid construct, it designates a coding sequence for the enzyme introduced into the host cell, the vector or the nucleic acid construct by genetic engineering. In the context of a host cell, it can mean that the coding sequence for the enzyme originates from a source different from the cell in which it is introduced. Alternatively, it can also mean that the coding sequence for the enzyme comes from the same species as the cell in which it is introduced but it is considered heterologous due to its environment which is not natural, for example because it is under the control of a promoter which is not its natural promoter, or is introduced at a location which differs from its natural location.

*Nucleic acid construct:* The term "nucleic acid construct" means a nucleic acid molecule, either single- or double-stranded, which is modified to contain segments of nucleic acids in a manner that would not otherwise exist in nature or which is synthetic, which comprises one or more control sequences.

*Operably linked:* The term "operably linked" means a configuration in which a control sequence is placed at an appropriate position relative to a coding sequence, in such a way that the control sequence directs expression of the coding sequence.

*Amino acid modifications or changes:* as used herein, by *"amino acid modification"* is meant a change in the amino acid sequence of a polypeptide. "Amino acid modifications" which may be also termed "amino acid changes", herein include amino acid mutations such as substitution, insertion, and/or deletion in a polypeptide sequence. By "amino acid substitution" or "substitution" herein is meant the replacement of an amino acid at a particular position in a parent polypeptide sequence with another amino acid. By "amino acid insertion" or "insertion" is meant the addition of an amino acid at a particular position in a parent polypeptide sequence. By "amino acid deletion" or "deletion" is meant the removal of an amino acid at a particular position in a parent polypeptide sequence.

*Parent enzyme or polypeptide:* as used herein, it is meant an unmodified enzyme that is subsequently modified to generate a variant. In the context of the invention, the parent enzyme may be a naturally-occurring α-ketoglutarate-dependent dioxygenase which has L-lysine and/or L-ornithine hydroxylase activity.

*Variant:* as used herein, a variant refers to a polypeptide sequence that differs from that of a parent polypeptide sequence by virtue of at least one amino acid modification. In the context of the invention, a variant derives from α-ketoglutarate-dependent dioxygenase, e.g. from a naturally-occurring α-ketoglutarate-dependent dioxygenase, and displays L-lysine and/or L-ornithine hydroxylase activity. Typically, a variant comprises from 1 to 50 amino acid modifications, preferably from 1 to 40 amino acid modifications. In particular, the variant may have from 1 to 20 amino acid changes, e.g. 1, 2, 3, 4, 5, 6, 7, 8, 9, 10, 11, 12, 13, 14, 15, 16, 17, 18, 19, or 20 amino acid modifications as compared to its parent. The sequence of a variant may comprise one or several amino acid substitutions, and/or, one or several amino acid insertions, and/or one or several amino acid deletions as compared to the sequence of its parent. In some embodiments, the amino acid modifications are conservative, preferably conservative substitutions. In other words, the amino acid modifications present in the variant do not significantly change its properties as compared to its parent. Conservative substitutions and the corresponding rules are well-described in the state of the art. Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill (1979, In, The Proteins, Academic Press, New York). Common substitutions are the followings Ala/Ser, Val/lle, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, LeuA al, Ala/Glu, and Asp/Gly. Alternatively, the amino acid modifications are of such a nature that the physico-chemical properties of the polypeptides are altered. For example, amino acid modifications may improve the thermal stability of the polypeptide, alter the substrate specificity, change the pH optimum, and the like. Essential amino acids in a polypeptide can be identified according to procedures known in the art, such as site-directed mutagenesis or alanine-scanning mutagenesis (Cunningham and Wells, 1989, Science 244: 1081 -1085). In the latter technique, single alanine mutations are introduced at every residue in the molecule, and the resultant mutants are tested for the capacity to hydroxylate L-lysine and/or to hydroxylate L-ornithine so as to identify amino acid residues that are critical to the L-lysine or L-ornithine hydroxylase activity. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for instance, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide. Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145). Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

*Sequence identity:* The sequence identity between two amino acid sequences is described by the parameter "percentage of identity". For purposes of the present invention, the "percentage of identity" between two amino acid sequences (A) and (B) is determined by comparing the two sequences aligned in an optimal manner, through a window of comparison. Said alignment of sequences can be carried out by well-known methods, for instance, using the algorithm for global alignment of Needleman-Wunsch. Protein analysis software matches similar sequences using measures of similarity assigned to various substitutions, deletions and other modifications, including conservative amino acid substitutions. Once the total alignment is obtained, the percentage of identity can be obtained by dividing the full number of identical amino acid residues aligned by the full number of residues contained in the longest sequence between the sequence (A) and (B). Sequence identity is typically determined using sequence analysis software. For comparing two amino acid sequences, one may use, for example, the tool "Emboss needle" for pairwise sequence alignment of proteins providing by EMBL-EBI and available on
www.ebi.ac.uk/Tools/services/web/toolform.ebi?tool=emboss_needle&context=protein, using default settings : (I) Matrix : BLOSUM62, (ii) Gap open : 10, (iii) gap extend : 0.5, (iv) output format : pair, (v) end gap penalty : false, (vi) end gap open : 10, (vii) end gap extend : 0.5.

### ■ Enzymes of the invention

In a first aspect, the invention relates to an enzyme having a L-lysine or L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:4, SEQ ID NO:9 and SEQ ID NO: 10, preferably from the group consisting of SEQ ID NO:1, SEQ ID NO:2, SEQ ID NO:3, and SEQ ID NO:4. As used herein, a first amino acid sequence having at least 70% of sequence identity with a second amino acid sequence encompasses an amino acid sequence having at least 70%, 75%, 80%, 85%, 90%, 93%, 95%, 96%, 97%, 98%, 99%, or 99.5% of sequence identity with said second amino acid sequence.

In some embodiments, the enzyme of the invention comprises or consists of an amino acid sequence having at least 80%, 85%, 90%, 93%, 95%, 96%, 97%, 98%, 99%, or 99.5% of sequence identity with an amino acid sequence selected from the group consisting of SEQ ID NO:1, SEQ ID NO:2, SEQ ID NO:3,SEQ ID NO:4, SEQ ID NO:9 and SEQ ID NO: 10.
The enzyme of the invention is preferably isolated and/or recombinant.

Furthermore, the enzyme of the invention may be a "α-ketoglutarate-dependent dioxygenase", this means that the enzyme of the invention may require the presence of a source of Fe²⁺ and a co-substrate, preferably an α-keto acid such as α-ketoglutaric acid or a salt thereof, in order to hydroxylate L-lysine and/or L-ornithine *in vitro* from molecular O₂.

In some embodiments, an enzyme of the invention may have both L-lysine hydroxylase activity and L-ornithine hydroxylase activity. In some other embodiments, an enzyme of the invention may have one activity selected among L-lysine hydroxylase activity and L-ornithine hydroxylase activity. L-ornithine hydroxylase activity and L-lysine hydroxylase activity of the enzyme of the invention may be assessed as described above in "Definition section".

More precisely, an enzyme of the invention is able to catalyze *in vitro* at least one of the reactions covered by the below general scheme:

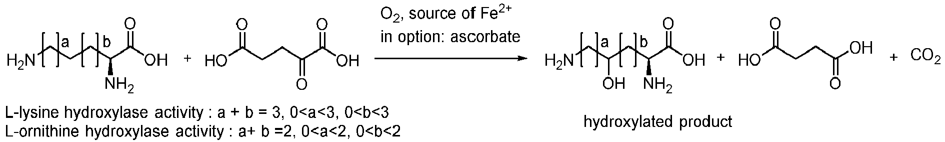

In some preferred embodiments, the enzyme of the invention is capable for hydroxylating L-lysine and/or L-ornithine in a regioselective manner. This means that the enzyme of the invention catalyzes the introduction of the hydroxyl group in a preferred position of the side-chain of L-lysine and/or L-ornithine. In other words, the hydroxylated product resulting from the reaction of the substrate (L-lysine or L-ornithine) with the enzyme of the invention comprises more than 80%, preferably more than 90%, 95%, 97%, 98%, or 99% of one specific regioisomer, the percentages being molar percentages.

In some further embodiments, the hydroxylation mediated by the enzyme of the invention is stereoselective. This means that one single diastereosiomer is preferably formed during the enzymatic reaction. In other words, more than 80%, preferably more than 90% and even more preferably more than 95%, 96%, 97%, 98% or 99% of the hydroxylated L-lysine or L-ornithine obtained with the enzyme of the invention corresponds to a single diastereoisomer. In some other embodiments, the enzyme of the invention hydroxylates the substrate (e.g. L-lysine or L-ornithine) with controlled regioselectivity and stereochemistry. In other words, the hydroxylated L-lysine or L-ornithine obtained with an enzyme of the invention essentially consists in a single diastereoisomer.

In some embodiments, the enzyme of the invention may display at least one of the following hydroxylase activities:
- the enzyme of the invention is capable of producing (3S)-hydroxy-L-lysine from L-lysine, and/or
- the enzyme of the invention is capable of producing (4R)-hydroxy-L-lysine from L-lysine, and/or
- the enzyme of the invention is capable of producing (3S)-hydroxy-L-ornithine from L-ornithine.

In some more additional aspects, the enzyme of the invention is enantiospecific, this means that the enzyme is capable of hydroxylating L-ornithine or L-lysine while being inefficient to hydroxylate D-lysine or D-ornithine and, preferably, in a more general aspect, to hydroxylate D-amino acids.

The enzyme of the invention may display an additional hydroxylase activity: for instance, the enzyme of the invention may be able to hydroxylate amino acids other than L-lysine and L-ornithine. In some embodiments, the enzyme of the invention is further able to hydroxylate a hydroxy-L-lysine. Such hydroxylation activities may be regioselective and/or stereoselective. As used herein, hydroxy-L-lysine encompasses 3-hydroxy-L-lysine, 4-hydroxy-L-lysine, and 5-hydroxy-L-lysine. A preferred hydroxy-L-lysine is 3-hydroxy-L-lysine or 5-hydroxy-L-lysine, in particular (5R)-hydroxy-L-lysine or (3S)-hydroxy-L-lysine.

As used herein, hydroxy-L-ornithine encompasses 3-hydroxy-L-ornithine, 4-hydroxy-L-ornithine, preferably 3-hydroxy-L-ornithine.

In some preferred embodiment, the enzyme of the invention is further able to hydroxylate at least one compound selected in the group consisting of (3S)-hydroxy-L-lysine, (5S)-hydroxy-L-lysine, (3R)-hydroxy-L-lysine, (5R)-hydroxy-L-lysine, and L-arginine, preferably at least one compound selected from the group consisting of (3S)-hydroxy-L-lysine (5R)-hydroxy-L-lysine and L-arginine.

In some other aspects, the enzyme of the invention is capable of hydroxylating L-lysine and 5-hydroxy-L-lysine, e.g. (5R)-hydroxy-L-lysine, and comprises an amino acid sequence having at least 70%, preferably at least 80%, of sequence identity with an amino acid sequence selected from the group consisting of SEQ ID NO:1, SEQ ID NO:2, SEQ ID NO:3. In another aspect, the enzyme of the invention is capable of hydroxylating L-lysine, 3-hydroxy-L-lysine, e.g. (3S)-hydroxy-L-lysine, and 5-hydroxy-L-lysine, e.g. (5R)-hydroxy-L-lysine and comprises an amino acid sequence having at least 70%, preferably at least 80%, of sequence identity with SEQ ID NO:2 or SEQ ID NO:3. In a more specific aspect, said enzyme may be further capable of oxidizing thia-L-lysine.

In certain aspects, the enzyme of the invention is capable of hydroxylating L-ornithine and comprises an amino acid sequence having at least 70%, preferably at least 80%, of sequence identity with SEQ ID NO:4. In a more specific aspect, said enzyme may be further capable of hydroxylating L-arginine. In some other or additional embodiments, the enzyme of the invention may be able to hydroxylate a hydroxy-L-ornithine.

In another aspect, the enzyme of the invention is capable of producing (3S)-hydroxy-L-lysine from L-lysine and comprises an amino acid sequence having at least 70%, preferably 80% of sequence identity with SEQ ID NO: 1. Said enzyme may be further capable of hydroxylating (5R)-hydroxy-L-lysine, for example into (3S,SR)-dihydroxy-L-lysine.

In an additional aspect, the enzyme of the invention is capable of producing (4R)-hydroxy-L-lysine from L-lysine and comprising an amino acid sequence having at least 70%, preferably 80% of sequence identity with an amino acid sequence selected from SEQ ID NO:2, SEQ ID NO:3, SEQ ID NO:9 and SEQ ID NO:10. Said enzyme may be further capable of hydroxylating (5R)-hydroxy-L-lysine, and/or (3S)-hydroxy-L-lysine. For instance, said enzyme may be capable of hydroxylating (5R)-hydroxy-L-lysine into (4S,5S)-dihydroxy-L-lysine and/or (3S)-hydroxy-L-lysine into (3R,4R)-dihydroxy-L-lysine.

In another aspect, the enzyme of the invention is capable of producing (3S)-hydroxy-L-ornithine from L-ornithine and comprising an amino acid sequence having at least 70%; preferably 80% of sequence identity with SEQ ID NO:4.

In a further aspect, the invention relates to an enzyme comprising an amino acid sequence having at least 70% of sequence identity SEQ ID NO:4 and having both L-ornithine and L-arginine hydroxylase activity. In some specific embodiments, the enzyme of the invention is able to hydroxylate L-ornithine and L-arginine at position C-3 of the side chains. For instance, the enzyme of the invention may be capable for converting L-arginine into (3S)-hydroxy-L-arginine and L-ornithine into (3S)-hydroxy-L-ornithine.

The enzyme of the invention may be a wild-type isolated enzyme, namely an isolated naturally-occurring enzyme, a variant of a wild-type enzyme, or a hybrid polypeptide.

In some embodiments, the enzyme of the invention is a wild-type enzyme isolated from a microorganism, especially from a bacterium.

Based on the teaching of the present disclosure, the one skilled in the art can identify other enzymes from microorganisms having L-lysine hydroxylase activity and/or L-ornithine hydroxylase activity as well as the other hydroxylase activities described here above. The polypeptide may be identified and obtained from microorganisms isolated from nature (e.g., soil, composts, water, etc.). Techniques for isolating microorganisms directly from natural habitats are well known in the art. Alternatively, a polynucleotide encoding an enzyme of the invention may be obtained by screening a genomic DNA, cDNA library from microorganisms, a mixed DNA sample or DNA samples obtained directly from natural materials (e.g., soil, composts, water, etc.). Once a polynucleotide encoding an enzyme of the invention has been detected, the polynucleotide can be isolated or cloned by utilizing techniques that are known to those of ordinary skill in the art.

For instance, the enzyme of the invention may be a wild-type enzyme isolated from a microorganism which belongs to a genus selected from *Flavobacterium, Chitinophaga, Niastella* and *Catenulispora.* Examples of appropriate microorganisms include, without being limited to, *Flavobacterium johnsoniae, Chitinophaga pinensis, Niastella koreensis,* and *Catenulispora acidiphila.*

In some other embodiments, the enzyme of the invention is a variant of a wild-type enzyme. Said wild-type enzyme comprises an amino acid sequence preferably selected from the group consisting of SEQ ID NO:1, SEQ ID NO:2, SEQ ID NO:3, SEQ ID NO:4, SEQ ID NO:9 and SEQ ID NO: 10.

In some preferred embodiments, the variant has an amino acid sequence which differs from an amino acid sequence selected from SEQ ID NO:1, SEQ ID NO:2, SEQ ID NO:3, SEQ ID NO:4, SEQ ID NO:9 or SEQ ID NO:10 in virtue of 1 to 40 amino acid modifications, preferably from 1 to 20 amino acid modifications, namely by 1, 2, 3, 4, 5, 6, 7, 8, 9, 10, 11, 12, 13, 14, 15, 16, 17, 18, 19 or 20 amino acid modifications. As mentioned above in the "definition section", amino acid modifications encompass insertion, deletion and substitution. In some embodiments, the amino acid modifications are conservative whereby the properties of the mutant are similar to those of its parent. In some other embodiments, the variant displays modified properties as compared to its parent enzyme, for example a modified solubility in aqueous medium and/or in solvent medium, an improved stability to pH, temperature and/or organic solvent, an improved L-lysine or L-ornithine hydroxylase activity and/or a modified regioselectivity or stereoselectivity as compared to its parent enzyme.

It goes without saying that a variant of the invention displays L-lysine and/or L-ornithine hydroxylase activity.

The methods for obtaining variants of a given enzyme are well-known in the art. Some of them are cited herein in the "Definition" section.

In some further embodiments, the enzyme of the invention is a hybrid polypeptide. As used herein, a hybrid polypeptide comprises an amino acid sequence having at least 70% of sequence identity with an amino acid sequence selected from the group consisting of SEQ ID NO:1, SEQ ID NO:2, SEQ ID NO:3 SEQ ID NO:4, SEQ ID NO:9 and SEQ ID NO:10, said amino acid sequence being fused or conjugated to another chemical or biochemical entity, preferably to another peptide or polypeptide. Accordingly, hybrid polypeptides of the invention thus encompass fused polypeptides and conjugated polypeptides. It goes without saying that said fused or conjugated polypeptide of the invention display one or several enzymatic activities as fully described here-above, in particular displays L-lysine and/or L-ornithine hydroxylase activity.

In some embodiments, the enzyme of the invention is a fused polypeptide in which the amino acid sequence as defined above is fused at its N-terminus or C-terminus region to another polypeptide region. Techniques for producing fusion polypeptides are known in the art, and include ligating the coding sequence encoding the enzyme and that encoding the other polypeptide polypeptide so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

The additional polypeptide region of the fusion polypeptide can be selected in order to enhance the stability of the enzyme according to the present disclosure, to promote the secretion (such as a N-terminal hydrophobic signal peptide) of the fusion polypeptide from a cell (such as a bacterial cell or a yeast cell), or to assist in the purification of the fusion polypeptide. More particularly, the additional region can be a tag useful for purification or immobilization of the enzyme. Such a tag is well-known by the person skilled in the art, for instance a His tag (His₆), a FLAG tag, a HA tag (epitope derived from the Influenza protein haemagglutinin), a maltose-binding protein (MPB), a MYC tag (epitope derived from the human proto-oncoprotein MYC), streptavidin or avidin, or a GST tag (small glutathione-S-transferase). A fusion polypeptide can further comprise a cleavage site between the enzyme and the addition region. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

Conjugated polypeptides refer to polypeptides wherein the amino acid sequence of the invention has been conjugated by chemical means to at least one chemical or biochemical entity. Techniques for conjugating an amino acid sequence to another chemical or biochemical entity are well-known in the art. The additional entity and the amino acid sequence of the invention may be covalently linked to each other directly or via a spacer. The spacer can be any standard linker commonly used for the preparation of polypeptide constructs. In some embodiments, the linker is a polypeptides comprising from 1 to 50 amino acid residues. Some preferred examples are Gly-Ser linkers such as tetraglycyl-seryl-triglycyl-serine peptide or polyalanine linkers.

The additional chemical or biochemical entities may be of any type. For instance, the additional or biochemical entities may be a mean useful for immobilizing the enzyme, e.g. a biotine or a reactive functional group, a mean for detecting the enzyme, a label and the like.

### ■ Nucleic acid constructs of the invention

The present invention relates to a polynucleotide encoding an enzyme of the present invention. The nucleic acid can be DNA (cDNA or gDNA), RNA, or a mixture of the two. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis.

A polynucleotide encoding an enzyme of the invention may be selected from the group consisting of:
- a nucleic acid that encodes an enzyme having a L-lysine or L-ornithine hydroxylase activity and comprising or consisting in an amino acid sequence having at least 80%, preferably at least 85%, 90%, 93%, 95%, 96%, 97%, 98%, 99%, or 99.5%, of sequence identity with an amino acid sequence selected from the group consisting of SEQ ID NO:1, SEQ ID NO:2, SEQ ID NO:3, SEQ ID NO:4, SEQ ID NO:9 and SEQ ID NO:10,
- a nucleic acid that encodes for a variant of an enzyme of amino acid sequence selected from SEQ ID NO:1, SEQ ID NO:2, SEQ ID NO:3, SEQ ID NO:4, SEQ ID NO:9 and SEQ ID NO: 10, said variant a L-lysine or L-ornithine hydroxylase activity, and
- a nucleic acid comprising a sequence selected from SEQ ID NO:5, SEQ ID NO:6, SEQ ID NO:7, SEQ ID NO:8, SEQ ID NO:13, SEQ ID NO:14 and an optimized version thereof.

The present invention also relates to nucleic acid constructs comprising a polynucleotide encoding an enzyme according to the present disclosure operably linked to one or more control sequences that direct the expression of the coding sequence in a suitable host cell under conditions compatible with the control sequences.

In some embodiments, the control sequence(s) is/are heterologous to the polynucleotide encoding the enzyme of the invention.

A polynucleotide may be manipulated in a variety of ways to provide for expression of the enzyme. Manipulation of the polynucleotide prior to its insertion into a vector may be desirable or necessary depending on the expression vector. The techniques for modifying polynucleotides utilizing recombinant DNA methods are well known in the art.

The control sequence may include a promoter that is recognized by a host cell or an *in vitro* expression system for expression of a polynucleotide encoding an enzyme of the present invention. The promoter contains transcriptional control sequences that mediate the expression of the enzyme. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters in a bacterial host cell are the promoters obtained from the *Bacillus amyloliquefaciens* alpha-amylase gene (amyQ), *Bacillus licheniformis* alpha-amylase gene (amyL), *Bacillus licheniformis* penicillinase gene (penP), *Bacillus stearothermophilus* maltogenic amylase gene (amyM), *Bacillus subtilis* levansucrase gene (sacB), *Bacillus subtilis* xylA and xylB genes, *Bacillus thuringiensis* crylllA gene (Agaisse and Lereclus, 1994, Molecular Microbiology 13: 97-107), *E. coli* lac operon, *E. coli* trc promoter (Egon et al., 1988, Gene 69: 301 -315), *Streptomyces coelicolor* agarase gene (dagA), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21 -25). Further promoters are described in "Useful proteins from recombinant bacteria" in Gilbert et al., 1980, Scientific American 242: 74-94; and in Sambrook et al., 1989. Examples of tandem promoters are disclosed in WO 99/43835.

Examples of suitable promoters in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (glaA), *Aspergillus oryzae* TAKA amylase, *Aspergillus oryzae* alkaline protease, *Aspergillus oryzae* triose phosphate isomerase, *Fusarium oxysporum* trypsin-like protease (WO96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene; and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (aprH), *Bacillus licheniformis* alpha-amylase (amyL), and *Escherichia coli* ribosomal RNA (rrnB).

Preferred terminators for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992, supra.

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis* crylllA gene (WO 94/25612) and a *Bacillus subtilis* SP82 gene (Hue et ai, 1995, Journal of Bacteriology 177: 3465-3471).

The control sequence may also be a leader, a non-translated region of an mRNA that is important for translation by the host cell. The leader is operably linked to the 5'-terminus of the polynucleotide encoding the enzyme. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide encoding the enzyme and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of the enzyme and directs the enzyme into the cell's secretory pathway. The 5'-end of the coding sequence of the polynucleotide may inherently contain a signal peptide coding sequence naturally linked in translation reading frame with the segment of the coding sequence that encodes the enzyme. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used. Effective signal peptide coding sequences for bacterial host cells are the signal peptide coding sequences obtained from the genes for *Bacillus* NCIB 1 1837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos et al., 1992, supra.

It may also be desirable to add regulatory sequences that regulate expression of the polypeptide relative to the growth of the host cell. Examples of regulatory systems are those that cause expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked with the regulatory sequence.

### Expression vectors

The present invention also relates to recombinant expression vectors comprising a nucleic acid construct as disclosed above, or a polynucleotide encoding an enzyme of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the enzyme at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extra-chromosomal entity, the replication of which is independent of chromosomal replication, e.g., a plasmid, an extra-chromosomal element, a mini-chromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophy, and the like.

Examples of bacterial selectable markers are *Bacillus licheniformis* or *Bacillus subtilis* genes or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, amdS (acetamidase), argB (ornithine carbamoyltransferase), bar (phosphinothricin acetyltransferase), hph (hygromycin phosphotransferase), niaD (nitrate reductase), pyrG (orotidine-5'-phosphate decarboxylase), and trpC (anthranilate synthase), as well as equivalents thereof. Preferred for use in an *Aspergillus* cell are *Aspergillus nidulans* or *Aspergillus oryzae* amdS and pyrG genes and a *Streptomyces hygroscopicus* gene.

The vector preferably contains an element(s) that permits integration of the vector into the host cell's genome or autonomous replication of the vector in the cell independent of the genome.

When integration into the host cell genome occurs, integration of the sequences into the genome may rely on homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo. Examples of bacterial origins of replication are the origins of replication of plasmids pBR322, pUC19, pACYC177, and pACYC184 permitting replication in *E*. *coli,* and pUB1 10, pE194, pTA1060, and pAMβ1 permitting replication in Bacillus. Examples of origins of replication for use in a yeast host cell are the 2 micron origin of replication, ARS1, ARS4, the combination of ARS1 and CEN3, and the combination of ARS4 and CEN6. Examples of origins of replication useful in a filamentous fungal cell are AMA1 and ANSI (Gems et al., 1991, Gene 98: 61 -67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

### Host cells

The present invention also relates to recombinant host cells, comprising a polynucleotide encoding the enzyme according to the present disclosure operably linked to one or more control sequences that direct the production of the enzyme of the present invention. A construct or vector comprising a polynucleotide encoding the enzyme of according to the present disclosure is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell depends upon the gene encoding the polypeptide and its source.

The host cell may be any cell useful in the recombinant production of a polypeptide of the present invention, e.g., a prokaryote or a eukaryote.

The prokaryotic host cell may be any Gram-positive or Gram-negative bacterium. Gram-positive bacteria include, but are not limited to, *Bacillus, Clostridium, Corynebacterium, Enterococcus, Geobacillus, Lactobacillus, Lactococcus, Oceanobacillus, Staphylococcus, Streptococcus,* and *Streptomyces.* Gram-negative bacteria include, but are not limited to, *Acinetobacter, Campylobacter, E. coli, Flavobacterium, Fusobacterium, Helicobacter, Ilyobacter, Neisseria, Pseudomonas, Salmonella,* and *Ureaplasma.* The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. The bacterial host cell may also be any *Streptococcus* cell including, but not limited to, *Streptococcus equisimilis, Streptococcus pyogenes, Streptococcus uberis, Streptococcus equi* and *Streptococcus zooepidemicus* cells. The bacterial host cell may further be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, e.g., Chang and Cohen, 1979, Mol. Gen. Genet. 168: 1 1 1 -1 15), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, e.g., Shigekawa and Dower, 1988, Biotechniques 6: 742-751), or conjugation (see, e.g., Koehler and Thorne, 1987, J. Bacteriol. 169: 5271 -5278). The introduction of DNA into an *E. coli* cell may be effected by protoplast transformation (see, e.g., Hanahan, 1983, J. Mol. Biol. 166: 557-580) or electroporation (see, e.g., Dower et al, 1988, Nucleic Acids Res. 16: 6127-6145). The introduction of DNA into a *Streptomyces* cell may be effected by protoplast transformation, electroporation (see, e.g., Gong et al., 2004, Folia Microbiol. (Praha) 49: 399-405), conjugation (see, e.g., Mazodier et al., 1989, J. Bacteriol. 171: 3583-3585), or transduction (see, e.g., Burke et al., 2001, Proc. Natl. Acad. Sci. USA 98: 6289-6294). The introduction of DNA into a *Pseudomonas* cell may be effected by electroporation (see, e.g., Choi et al., 2006, J. Microbiol. Methods 64: 391 -397) or conjugation (see, e.g., Pinedo and Smets, 2005, Appl. Environ. Microbiol. 71: 51 -57). The introduction of DNA into a *Streptococcus* cell may be effected by natural competence (see, e.g., Perry and Kuramitsu, 1981, Infect. Immun. 32: 1295-1297), protoplast transformation (see, e.g., Catt and Jollick, 1991, Microbios 68: 189-207), electroporation (see, e.g., Buckley et al., 1999, Appl. Environ. Microbiol. 65: 3800-3804), or conjugation (see, e.g., Clewell, 1981, Microbiol. Rev. 45: 409-436). However, any method known in the art for introducing DNA into a host cell can be used.

The host cell may also be a eukaryote, such as a mammalian, insect, plant, or fungal cell, in particular yeast cell. The host cell may be a fungal cell. "Fungi" as used herein includes the phyla *Ascomycota, Basidiomycota, Chytridiomycota,* and *Zygomycota* as well as the *Oomycota* and all mitosporic fungi (as defined by Hawksworth et al., In, Ainsworth and Bisby's Dictionary of The Fungi, 8th edition, 1995, CAB International, University Press, Cambridge, UK). The fungal host cell may be a yeast cell. "Yeast" as used herein includes ascosporogenous yeast (Endomycetales), basidiosporogenous yeast, and yeast belonging to the *Fungi imperfecti* (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980). The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell. The fungal host cell may be a filamentous fungal cell. "Filamentous fungi" include all filamentous forms of the subdivision *Eumycota* and *Oomycota* (as defined by Hawksworth et al., 1995, supra). The filamentous fungi are generally characterized by a mycelial wall composed of chitin, cellulose, glucan, chitosan, mannan, and other complex polysaccharides. The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell. For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known per se. Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

The cell can also be a mammalian cell, for example COS, CHO (US 4,889,803; US 5,047,335). In a particular embodiment, the cell is non-human and non-embryonic. In addition, the enzyme of the invention could be produce by a non-human transgenic animal, for instance in the milk produces by the animal.

The cell can be a plant cell. Then, the enzyme of the invention could be produce by a transgenic plant.

Preferred strains are *E. coli* and *Corynebacterium glutamicum*

Host cells of particular interest in the present disclosure are host cells which overproduce L-lysine, L-ornithine or α-ketoglutaric acid. Several microorganisms are known to, or have been modified so as to, overproduce L-lysine, L-ornithine or α-ketoglutaric acid.

For overproducing L-lysine, one can cite strains from *Corynebacterium glutamicum* (Sahm et al, Annals of the New York Academy sciences, 1996, 782, 25-39), from *Saccharomyces cerevisiae* (Gasent-Ramirez, Applied and Environmental microbiology, 1997, 4800-4806), or from *Escherichia coli* (Imaizumi et al, J Biotechnol, 2005, 117,111-118). For review about this matter see Wittmann and Becker, Microbiol Monogr, 2007, DOI 10.1007/717_L2006_089), the disclosure of which being incorporated herein by reference. For strains from *Corynebacterium glutamicum* or *Escherichia coli* which overproduce L-ornithine, see for instance Jiang et al. BMC Biotechnology, 2013, 13:47, and Lee and Cho Biotechnol Lett, 2006, 28:1849-1856, the disclosure of which being incorporated by reference.

At last, strains overproducing α-ketoglutaric acid have been also described and encompass strains from *Corynebacterium glutamicum, Bacillus genus* and *Pseudomonas fluorescens.* For review about this matter see for instance Otto et al., Appl. Microbiol Biotechnol, 2011, 92:689-695, the disclosure of which being incorporated by reference.

Preferred strains which overproduce L-lysine, L-ornitine or α-ketoglutaric acid are genetically engineered host cells. Such strains may be prepared by standard metabolic and genetic engineering techniques as illustrated in Wittmann and Becker (see supra).

Accordingly, in some embodiments a strain of the invention is able to produce an enzyme of the invention and overproduces α-ketoglutaric acid and/or, L-lysine or L-ornithine.

The host cells of the invention may be used for producing the enzyme of the invention. Alternatively, the host cells of the invention may be used for implementing the synthesis methods of the invention, as described above.

### Method of production of the enzyme

The present invention also relates to methods for producing the enzyme of the present invention by using a cell which naturally produces the enzyme of the invention or by using a host cell according to the invention.

In a more specific aspect, the invention relates to a method for producing an enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3 SEQ ID NO:4, SEQ ID NO:9 and SEQ ID NO:10, said method comprising:
- cultivating a cell, which in its wild-type form is capable of producing the enzyme, or a recombinant host cell of the invention, in conditions conducive for production of the enzyme, and
- recovering and/or purifying the enzyme.

It goes without saying that the enzyme may have one or several features as fully-described in the above section entitled "enzymes of the invention".

The cell is cultivated in a nutrient medium suitable for production of the enzymes of the invention using methods known in the art. For example, the cell may be cultivated by shake flask cultivation, or small-scale or large-scale fermentation (including continuous, batch, fed-batch, or solid state fermentations) in laboratory or industrial fermentors performed in a suitable medium and under conditions allowing the enzyme to be expressed and/or isolated. The cultivation may take place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the enzyme is secreted into the nutrient medium, the enzyme can be recovered directly from the medium. If the enzyme is not secreted, it can be recovered from cell lysates. The enzyme may be detected using methods known in the art that are specific for the enzyme. These detection methods include, but are not limited to, use of specific antibodies, detection of tag, formation of an enzyme product, or disappearance of an enzyme substrate. For example, an enzyme assay may be used to determine the activity of the enzyme.

The enzyme may be recovered using methods known in the art. For example, the enzyme may be recovered from the nutrient medium by conventional procedures including, but not limited to, collection, centrifugation, filtration, extraction, spray-drying, evaporation, and/or precipitation.

The enzyme may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, Janson and Ryden, editors, VCH Publishers, New York, 1989) to obtain substantially pure enzyme. In an alternative aspect, the enzyme is not recovered, but rather a host cell of the present invention expressing the enzyme, or a lysate thereof, is used as a source of the enzyme.

### ■ Supports, compositions and kits of the invention

The enzyme of the invention may be immobilized on a support. The present invention thus relates to a support on which an enzyme of the invention is immobilized. The invention further relates to a method for preparing such a support comprising the step of providing an enzyme according to the invention and immobilizing said enzyme on a support. The enzyme of the invention may be immobilized by any appropriate method described in the state in the art, for instance, covalent binding, adsorption, entrapment or membrane confinement. It goes without saying that the enzyme of the invention still displays at least L-lysine or L-ornithine hydroxylase activity once immobilized.

A wide variety of supports may be used for immobilizing the enzyme of the invention. The support to select depends on its dedicated use. Convenient supports encompass, without being limited to, plastic, metal, inorganic support such as glass, silica, alumina, bentonite, hydroxyapatite, nickel/nickel oxide, titanium, zirconia, polymeric supports and the like. The support may be in the form of a surface, a powder, micro- or nanobeads, a gel, a solvent-swelling or water-swelling gel or matrix, a reticulated matrix or gel, a membrane, a fibrous support, a porous support and the like.

In a preferred embodiment, the support is selected among inorganic matrices and polymeric matrices. For instance, supports useful for the invention encompass resins or matrices comprising or consisting in polyoside such as cellulose, carboxymethylcellulose, diethylaminocellulose (DEAE), dextran, cross-linked dextran such as Sephadex®, agarose, cross-linked agarose such as Sepharose®, starches, alginate, chitosan, a synthetic polymer such as polyaminoacids, polyacrylamides, polymers and copolymers based on acrylic acid and derivatives thereof, polyamides, polystyrene, organopolysiloxanes, polyacrylate, polyvinyls polyacrilin, inorganic compounds such as hydroxyapatite, silica or bentonite, and the like. Such supports are commercially available (see for instance, Duolite®, Amberlite®, Sephadex®, Eupergit® resins).

The methods for immobilizing the enzyme of the invention are well-known to the skilled artisan (see for instance, Tischer and Wedekind, Topics in Current Chemistry, 1999, 200, 95-126 and Alloue et al, Biotechnol Agron Soc Environ 2008, 12, 57-68; the disclosure of which being incorporated herein by reference).

For instance, the enzyme may be entrapped in a polymeric matrix, for instance a matrix of alginate or chitosan. As an alternative, the enzyme of the invention may be covalently linked to the support. Typically, the support may contain functional groups able to react directly, or after activation, with an amino acid present in the enzyme of the invention so as to create a covalent link. For instance, the support may have thereon -COOH groups which can react upon activation with an amino group present in the side-chain of an amino acid residue of the enzyme, directly or by the means of a spacer or a bifunctional coupling agent. As an alternative, the enzyme of the invention may be absorbed on the support. The interactions between the support and the enzyme may be then stabilized by cross-linking with a bifunctional agent such as glutaraldehyde.

Once prepared, the support of the invention can be directly used in a reaction medium. In other words, the support of the invention may be merely added in the reaction medium. When the support is solvent-swelling, the solvent of the reaction may be selected so as to provide an appropriate swelling of the support to render accessible the immobilized enzyme without impairing the catalytic activity of the enzyme. As an alternative, the support can be used to prepare a reactor, which can be for instance an enzyme reactor, a membrane reactor, a continuous flow reactor such as a stirred tank reactor, a continuously operated packed bed reactor, or a continuously operated fluidized bed reactor, or a packed bed reactor.

In some embodiments, the support of the invention is recyclable and may be used several times in a row.

The enzyme of the invention can be also formulated in a composition. A further aspect of the invention is thus a composition comprising an enzyme of the invention and an excipient. The composition may be liquid or dry, for instance in the form of a powder. In some embodiments, the composition is a lyophilisate. The enzyme may be present in a purified or in an enriched form. Appropriate excipients encompass buffers commonly used in biochemistry, agents for adjusting pH, antioxidant, redox agent such as dithiothreitol, preservatives such as sodium benzoate, sodium sorbate or sodium ascorbate, conservatives, protective or stabilizing agents such as starch, malodextrin, arabic gum, salts, sugars e.g. sorbitol, trehalose or lactose, glycerol, polyethyleneglycol, polyethene glycol, polypropylene glycol, propylene glycol, sequestering agent such as EDTA, amino acids, a carrier such as a solvent or an aqueous solution, and the like. The composition of the invention may also comprise a source of Fe²⁺ such as FeSO₄ or α-ketoglutaric acid or a salt thereof.

The composition of the invention may comprises from 1% to 99%, preferably from 10% to 95% by weight of an enzyme of the invention and from 1% to 99%, preferably from 5% to 90% by weight of excipient(s).

The composition of the invention may be obtained by mixing the enzyme with one or several excipients.

For instance, when the composition is a powder, said composition may be obtained by freeze-drying or spray-drying. Typically, the enzyme of the invention is solubilized in an aqueous medium together with one or several excipients, especially excipients which are able to stabilize or protect the enzyme from degradation. Such excipients are for instance glycerol, sorbitol, lactose or trehalose which may be used in amount of 10% to 50% (w/v) in the solution. The resulting mixture is then freeze-dried or spray-dried so as to obtain a powder.

In a further aspect, the invention relates to a kit comprising:
- an enzyme of the invention, or
- a composition comprising an enzyme of the invention, or
- a support of the invention, or
- a host cell able to express an enzyme of the invention as described above.

Said kit is preferably dedicated for implementing a method of the invention such as those described hereunder, especially a method for hydroxylating a compound of formula (I) or a method for preparing hydroxylated L-lysine, hydroxylated L-ornithine or analogs thereof. The kit may further comprise:
- reagents such as a buffer, a source of Fe²⁺ e.g. FeCl₂ or FeSO₄, a α-ketoacid, for instance α-ketoglutaric acid, α-ketoadipic acid or a salt or a derivative thereof, ascorbic acid or a salt thereof, L-lysine or an analog thereof, L-ornithine or an analog thereof, and/or
- compounds required for culturing the host cell such as nutrients, a culture medium, a mean for assessing the growth of the host cell, and the like,
- a mean for detecting or quantifying the progress of the enzymatic reaction, and/or
- written instructions, for instance, relating to the running conditions for implementing the method, in particular the pH and the temperature to use.

The present invention also relates to a kit for preparing a support of the invention, said kit comprising an enzyme of the invention and at least one of the following items:
- a support for the immobilization of the enzyme, and/or
- a reagent for immobilizing the enzyme of the invention on said support and/or
- written instructions, for instance, relating to the running conditions for immobilizing the enzyme on the support.

### ■ Synthesis methods of the invention

### Method for hydroxylating a compound of formula (I)

In a further aspect, the invention relates to a method for hydroxylating a compound of formula (I), or a salt thereof: 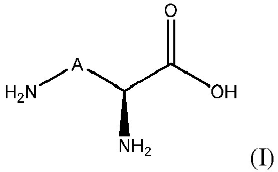 wherein
- A is -(CH₂)ₙ-X-(CH₂)ₚ- with X is CH₂, or CH(OH), n is an integer from 0 to 4, preferably from 1 to 4, and p is an integer from 0 to 4 with proviso that 0≤ n+p ≤ 4,
   wherein said method comprises
   - contacting the compound of formula (I) or a salt thereof with an enzyme having a L-lysine or L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:4; SEQ ID NO:9 and SEQ ID NO:10, preferably from the group consisting of SEQ ID NO:1, SEQ ID NO:2, SEQ ID NO:3, and SEQ ID NO:4, and
   - optionally recovering the hydroxylated compound.

The hydroxylated compound refers to the compound resulting from the hydroxylation of the compound of formula (I) with said enzyme of the invention. In other words, the method of the invention is for preparing a hydroxylated compound from a compound of formula (I) as described above.

Preferably, the method of the invention enables to introduce a hydroxyl group, preferably one single hydroxyl group, on a carbon position of moiety A of the compound of formula (I) or a salt thereof.

In some embodiments of the method of the invention, the compound of formula (I) is such that n and p are integers from 0 to 3 with proviso that (n + p) is equal to 2, 3 or 4.

In some other embodiments, the compound of formula (I) is such that n is an integer from 1 to 3 and p is an integer from 0 to 2 with proviso that (n + p) is equal to 2 or 3.

In some embodiments, the compound of formula (I) is such that n is 1, 2 or 3 and p is 0, 1, or 2 with the proviso that n+ p = 3.

In other embodiments, the compound of formula (I) is such that n is 1 or 2 and p is 0 or 1 with proviso that n+ p =2.

In some further embodiments, the method of the invention is for hydroxylating a compound of formula (I) selected from L-lysine, L-ornithine, a hydroxy-L-lysine, a hydroxy-L-ornithine. As used herein, hydroxy-L-lysine encompasses 3-hydroxy-L-lysine, 4-hydroxy-L-lysine, and 5-hydroxy-L-lysine. A preferred hydroxy-L-lysine is 3-hydroxy-L-lysine or 5-hydroxy-L-lysine, in particular (5R)-hydroxy-L-lysine or (3S)-hydroxy-L-lysine.

In a preferred embodiment, the method of the invention is for hydroxylating a compound of formula (I) selected from the group consisting of L-lysine, L-ornithine, 5-hydroxy-L-lysine, preferably (5R)-hydroxy-L-lysine, 3-hydroxy-L-Lysine, preferably (3S)-hydroxy-L-lysine, or a salt thereof.

In a further aspect, the method of the invention is for hydroxylating a compound of formula 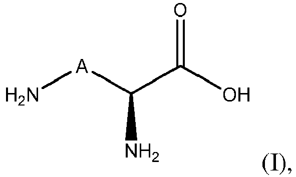 or a salt thereof:
wherein A is -(CH₂)ₙ-X-(CH₂)ₚ- with X is CH₂, or CH(OH), n is an integer from 1 to 3 and p is an integer from 0 to 2 with proviso that n + p =3
wherein said method comprises
- contacting the compound of formula (I) or a salt thereof with an enzyme having a L-lysine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9 and SEQ ID NO:10, preferably from the group consisting of SEQ ID NO:1, SEQ ID NO:2, SEQ ID NO:3; and
- optionally recovering the resulting hydroxylated compound.

Preferably, the hydroxyl group is introduced on a carbon position of moiety A of the compound of formula (I). In such embodiments, the compound of formula (I) is preferably L-lysine or a hydroxy-L-lysine. A preferred hydroxy-L-lysine is 3-hydroxy-L-lysine or 5-hydroxy-L-lysine, in particular (5R)-hydroxy-L-lysine or (3S)-hydroxy-L-lysine.

In an additional aspect, the method of the invention is for hydroxylating a compound of formula (I), or a salt thereof: 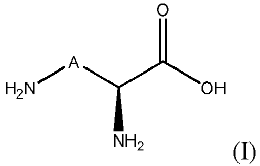 wherein A is -(CH₂)ₙ-X-(CH₂)ₚ- with X is CH₂, or CH(OH), n is 1 or 2, and p is 1 or 2 with proviso that n + p =3 wherein said method comprises
- contacting the compound of formula (I) or a salt thereof with an enzyme having a L-lysine hydroxylase activity and comprising an amino acid sequence having at least 70% of sequence identity with the amino acid sequence of SEQ ID NO:1, and
- optionally recovering the resulting hydroxylated compound.

In a more specific aspect, the compound of formula (I) is L-lysine, or 5-hydroxy-L-lysine in particular L-lysine or (5R)-hydroxy-L-lysine.

In another aspect, the method of the invention is for hydroxylating a compound of formula (I), or a salt thereof: 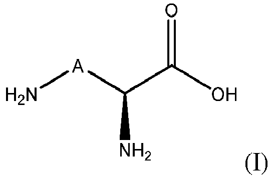 wherein A is -(CH₂)ₙ-X-(CH₂)ₚ- with X is CH₂, or CH(OH), n is 0, 1 or 3, and p is 0, 1 or 3, with proviso that n + p =3 wherein said method comprises
- contacting the compound of formula (I) or a salt thereof with an enzyme having a L-lysine hydroxylase activity and comprising an amino acid sequence having at least 70% of sequence identity with an amino acid sequence selected from SEQ ID NO:2 SEQ ID NO:3, SEQ ID NO:9, and SEQ ID NO: 10 and
- optionally recovering the resulting hydroxylated compound.

In a more specific aspect, the compound of formula (I) is L-lysine, 5-hydroxy-L-lysine or 3-hydroxy-L-lysine, in particular L-lysine, (5R)-hydroxy-L-lysine or (3S)-hydroxy-L-lysine.

In another aspect, the method of the invention is for hydroxylating a compound of formula (I), or a salt thereof: 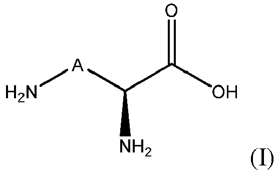 wherein A is -(CH₂)ₙ-X-(CH₂)ₚ- with X is CH₂, or CH(OH), and n is 0 or 1 and p is an integer from 1 or 2 with proviso that n + p =2 wherein said method comprises
- contacting the compound of formula (I) or a salt thereof with an enzyme having a L-ornithine hydroxylase activity and comprising an amino acid sequence having at least 70% of sequence identity with SEQ ID NO:4; and
- optionally recovering the resulting hydroxylated compound, and
wherein the hydroxyl group is introduced on a carbon position of moiety A.

In a preferred embodiment, the compound of formula (I) is L-ornithine.

In the above methods of the invention, the hydroxylation of compound of formula (I) is preferably regioselective and/or stereoselective: this means that the hydroxylated product resulting from the reaction of the compound of formula (I) with the enzyme of the invention mostly consists in a single diastereoisomer (since the hydroxylated product comprises at least two asymmetric carbon atoms). More precisely, said products comprise more than 80%, 90%, 95%, 96%, 97%, 98%, 99% by moles of a specific diastereoisomer, the percentage referring to the total molar amount of hydroxylated products formed by reaction of the compound of formula (I) with the enzyme.

In a further aspect, the method of the invention is for preparing (3S)-hydroxy-L-lysine, and comprises contacting L-lysine with an enzyme capable of producing (3S)-hydroxy-L-lysine from L-lysine and comprising an amino acid sequence having at least 70% of sequence identity with SEQ ID NO: 1.

In an alternate aspect, the method of the invention is for preparing (4R)-hydroxy-L-lysine, said method comprises contacting L-lysine with an enzyme capable of producing (4R)-hydroxy-L-lysine from L-lysine and comprising an amino acid sequence having at least 70% of sequence identity with an amino acid sequence selected from SEQ ID NO:2, SEQ ID NO:3, SEQ ID NO:9 and SEQ ID NO:10.

In another aspect, the method of the invention is for preparing (3S)-hydroxy-L-ornithine and comprises contacting L-ornithine with an enzyme capable of producing (3S)-hydroxy-L-ornithine from L-ornithine and comprising an amino acid sequence having at least 70% of sequence identity with SEQ ID NO:4.

In an alternate aspect, the method is for preparing a dihydroxy-L-lysine, said method comprising contacting a hydroxy-L-lysine, such as a 5-hydroxy-L-lysine, or 3-hydroxy-L-lysine, with an enzyme having a L-lysine hydroxylase activity and comprising an amino acid sequence having at least 70% of sequence identity with an amino acid sequence selected from SEQ ID NO:1, SEQ ID NO:2, SEQ ID NO:3, SEQ ID NO:9 and SEQ ID NO:10, preferably SEQ ID NO:1, SEQ ID NO:2, and SEQ ID NO:3.

Examples of dihydroxy-L-lysine which may be prepared by the method of the invention encompass, without being limited to:

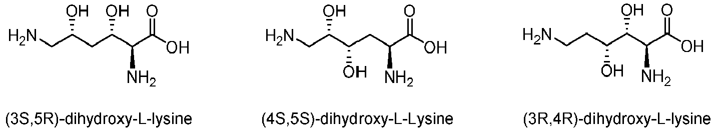

For instance, said method may comprise contacting a 5-hydroxy-L-lysine with an enzyme having a L-lysine hydroxylase activity and comprising an amino acid sequence having at least 70% of sequence identity with SEQ ID NO:1, whereby a 3,5-dihydroxy-L-lysine is obtained. In such an embodiment, (3S,5R)-dihydroxy-L-lysine may be obtained from (5R)-hydroxy-L-lysine.

Alternatively, said method may comprise contacting a 5-hydroxy-L-lysine with an enzyme having a L-lysine hydroxylase activity and comprising an amino acid sequence having at least 70% of sequence identity with SEQ ID NO:3 or SEQ ID NO:2, whereby a 4,5-dihydroxy-L-lysine is obtained. In such an embodiment, (4S,5S)-dihydroxy-L-lysine may be obtained from (5R)-hydroxy-L-lysine.

In another embodiment, said method may comprise contacting a 3-hydroxy-L-lysine with an enzyme having a L-lysine hydroxylase activity and comprising an amino acid sequence having at least 70% of sequence identity with SEQ ID NO:3 or SEQ ID NO:2, whereby a 3,4-dihydroxy-L-lysine is obtained. In such an embodiment, (3R,4R)-dihydroxy-L-lysine is obtained from (3S)-hydroxy-L-lysine.

It goes without saying that, in the synthesis methods of the invention, the step of contacting an enzyme of the invention with a compound of formula (I) is performed in conditions conducive for the enzymatic activity, namely the hydroxylase activity. For instance, the step of contacting the enzyme of the invention with the compound of formula (I) is preferably performed in the presence of an α-ketoacid or a salt thereof as co-substrate, and a source of Fe²⁺ as a co-factor.

Appropriate α-ketoacids and salts thereof encompass, without being limited to, α-glutaric acid, α-ketoadipic acid and salt thereof such as sodium or potassium salts.

Appropriate sources of Fe²⁺ encompass, without being limited to, Mohr's salt, ferrous sulfate or ferrous chloride.

Additional chemical substances for stabilizing the enzyme or for improving the enzymatic activity may be present in the reaction medium such as ascorbic acid and salts thereof, dithiothreitol or catalase.

At last, the step of contacting the enzyme with the compound of formula (I) is preferably performed in the presence of a source of molecular O₂ which may be merely atmospheric or produced *in situ* by an appropriate mean.

Preferred enzymes for implementing the method of the invention are those described herein in the section entitled "enzymes of the invention".

In the synthesis methods of the invention, the enzyme may be used in any appropriate forms, in particular those described herein. For instance, the enzyme is provided in a free state, for example in an isolated form, in an enriched form, in a purified form or in a semi-purified form. For instance, the enzyme of the invention may be present in a supernatant or in a supernatant extract recovered from a culture. The enzyme may be provided as a cell lysate. Alternatively, the enzyme may be formulated in a composition. In some embodiments, the enzyme is immobilized on a support as described herein. In some other embodiments, the enzyme may be expressed *in situ* by a host cell of the invention, or by a cell which is able to express in its wild-type form the enzyme of the invention.

In some embodiments, the method of the invention comprises:
(a) providing a product selected among the group consisting of an enzyme of the invention, a composition comprising an enzyme of the invention, a support on which an enzyme of the invention is immobilized, and a cell or a host cell able to express the enzyme of the invention or a cell lysate thereof,
(b) contacting the product provided in step (a) with the compound of formula (I) so as to promote the hydroxylation of compound of formula (I), and
(c) optionally recovering and/or purifying the resulting hydroxylated compound.

The running conditions may vary, among others, depending on the source of enzyme which is used. When the enzyme is used in a free state, the pH, the temperature and the solvent are selected so as to promote enzymatic activity. When the enzyme is expressed *in situ* by a cell or a host cell, the running conditions may be also conducive for the expression of the enzyme by said cell or host cell.

Host cells of interest are those described herein in the section entitled "host cell" and able to be cultured in aerobiosis. For instance, host cells for implementing the method of the invention may be mutants obtained from *E*. *coli.*

The method of the invention further comprises a step of recovering and/or purifying the resulting hydroxylated compound. Said compound may be recovered and/or purified from the reaction medium by any convenient method such as precipitation, filtration, extraction, liquid chromatography and combinations thereof.

Another object of the invention is the use of:
- an enzyme of the invention; or
- the solid support with an immobilized enzyme of the invention; or
- the host cell capable of expressing an enzyme or a cell lysate thereof; or
- a wild-type cell capable of expressing an enzyme of the invention, or a cell lysate thereof
   for hydroxylating a compound of formula (I): 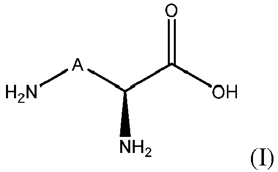
wherein A is -(CH₂)ₙ-X-(CH₂)ₚ- with X is CH₂, or CH(OH), n is an integer from 0 to 4, preferably from 1 to 4, and p is an integer from 0 to 4 with proviso that 0≤ n+p ≤ 4

Preferred uses according to the invention are for hydroxylating L-lysine, L-ornithine, and a hydroxy-L-lysine such as (5R)-hydroxy-L-lysine and (3S)-hydroxy-L-lysine.

In other word, preferred uses are for preparing a hydroxy-L-lysine, a hydroxy-L-ornithine or a dihydroxy-L-lysine such that (3S)-hydroxy-L-lysine, (4R)-hydroxy-L-lysine, (3S)-hydroxy-L-lysine, or (3R,4R)-dihydroxy-L-lysine, (3S,5R)-dihydroxy-L-lysine and (4S,5S)-dihydroxy-1-lysine.

Accordingly, another preferred object of the invention is the use of:
- an enzyme of the invention; or
- the solid support with an immobilized enzyme of the invention; or
- the host cell capable of expressing an enzyme or a cell lysate thereof; or
- a wild-type cell capable of expressing an enzyme of the invention or a cell lysate thereof;
for preparing a compound selected from (3S)-hydroxy-L-lysine, (4R)-hydroxy-L-lysine or (3S)-hydroxy-L-ornithine.

A further object of the invention, is the use of:
- an enzyme of the invention; or
- the solid support with an immobilized enzyme of the invention; or
- the host cell capable of expressing an enzyme; or
- a wild-type cell capable of expressing an enzyme of the invention;
for preparing a dihydroxy-L-lysine such as 3,4-dihydroxy-L-lysine, 4,5-dihydroxy-L-lysine or 3,5-dihydroxy-L-lysine.

In an additional aspect, the invention relates to the use of a hydroxylated compound obtained by the method according to the invention as building blocks or as a synthon for chemical synthesis. For instance, the resulting hydroxylated compound may be used as amino acid residue for the synthesis of peptides or peptidomimetic compounds.

### Method for preparing a compound of interest

The invention also relates to a process for preparing a compound of interest comprising:
- producing a hydroxylated compound from a compound of formula (I) or a salt thereof by the method according to the present invention and
- using the resulting hydroxylated compound or a salt thereof for preparing the compound of interest.

The compound of interest may be of any type. Said compounds may be an oligopeptide, a peptide, a peptidomimetic compound or any kind of organic molecules. The compound of interest may be a final product which may be used, for instance as a drug. Alternatively, the compound of interest may be a building block or a synthesis intermediate which may be used for the synthesis of other molecules.

The process of the invention is preferably diastereoselective, this means that said method enables to obtain the compound of the interest in an enriched-diastereoisomer form, which means that a single diastereoisomer of the compound of interest is mostly obtained. Said diastereoisomer may account for at least 80%, 90%, 95% , 99%, even 99.9% in moles of the resulting compound of interest.

For instance, the compound of interest may be 1,5-diamino-2-pentanol, e.g. (2S) -1,5-diamino-2-pentanol, 1,5-diamino-3-pentanol, 1,4-diamino-2-butanol e.g. (2S)-1,4-diamino-2-butanol, dihydroxy-L-lysine such as 3,4-dihydroxy-L-lysine, e.g. (3R,4R)-dihydroxy-L-lysine, 4,5-dihydroxy-L-lysine or 3,5-dihydroxy-L-lysine and a 1,5-diamino-pentane-diol such as 1,5-diamino-pentane-2,3-diol, e.g. (2R,3R)-1,5-diaminopentane-2,3-diol.

In some embodiments, the compound of interest may be a hydroxylated diamino compound, in particular a hydroxylated diamino alkane.

### Method for preparing a hydroxylated diaminoalkane

The invention also relates to a method for preparing a hydroxylated diamino alkane (also called herein diamino alkanol). The resulting hydroxylated diamino alkanes may be used as building blocks or as synthetic intermediates for preparing more complex molecules, in particular in the pharmaceutical field.

The method for preparing a hydroxylated diamino alkane comprises:
(i) providing a compound of formula (I): 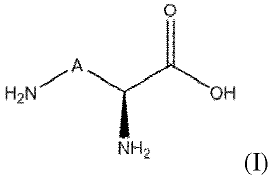 wherein A is -(CH₂)ₙ-X-(CH₂)ₚ- with X is CH₂, or CH(OH) and n is an integer from 0 to 4, preferably from 1 to 4, and p is an integer from 0 to 4 with proviso that 0≤ n+p ≤ 4, preferably n+p = 2 or 3,
(ii) hydroxylating compound of formula (I) by contacting said compound with an enzyme having a L-lysine or L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:4, SEQ ID NO:9 and SEQ ID NO:10,
(iii) decarboxylating the compound obtained in step (I), and
(iv) optionally recovering the resulting hydroxylated diamino alkane.

Typically, the step (ii) corresponds to the method for hydroxylating a compound of formula (I) according to the invention, as fully described above. Preferred embodiments of step (ii) thus correlate with those disclosed herein for the method for hydroxylating a compound of formula (I) according to the invention.

The step (iii) of decarboxylating may be performed by any method known by the skilled artisan. In a preferred embodiment, step (iii) is performed by reacting the hydroxylated compound obtained in step (ii) with a decarboxylase.

As used herein, a decarboxylase (also called carboxy-lyase) refers to an enzyme belonging to EC 4.1.1 which is able to catalyze the removal of a -COOH group from an organic compound, in particular from an amino acid. Preferred decarboxylases are pyridoxal phosphate (PLP)-dependent carboxylases and in particular amino acid PLP-dependent decarboxylases such as L-ornithine decarboxylases (EC 4.1.1.17) and L-lysine decarboxylases (EC 4.1.1.18).

For review concerning PLP-dependent decarboxylases, please refer to Jordan and Patel, ACS Catal., 2013,3, 1601-1617, the disclosure of which being incorporated by reference.

In some specific embodiments, the pyridoxal-dependent decarboxylase may be a naturally-occurring isolated pyridoxal-dependent decarboxylase from a microorganism which belongs to a genus selected from *Flavobacterium, Chitinophaga, Niastella* and *Catenulispora,* or may be a variant thereof. Examples of appropriate microorganisms include, without being limited to, *Flavobacterium johnsoniae, Chitinophaga pinensis, Niastella koreensis,* and *Catenulispora acidiphila.*

In some further specific embodiments, step (iii) is performed by reacting the hydroxylated compound obtained in step (ii) with a pyridoxal dependent decarboxylase comprising an amino acid sequence having a sequence identity of at least 70%, with the amino acid sequence of SEQ ID NO:11 or SEQ ID NO: 12.

In some embodiments, the pyridoxal-dependent decarboxylase used in step (iii) comprises an amino acid sequence having at least 80%, 85%, 90%, 95% or 99% of sequence identity with SEQ ID NO:11 or SEQ ID NO: 12.

It goes without saying that in step (iii), the pyridoxal-dependent decarboxylase may be used in any form known by the skilled artisan. The pyridoxal-dependent decarboxylase may be provided in a free state, for example in an isolated form, in an enriched form, in a purified form or in a semi-purified form. For instance, the pyridoxal-dependent decarboxylase may be present in a supernatant or in a supernatant extract recovered from a culture. The enzyme may be provided as a cell lysate. Alternatively, the pyridoxal-dependent decarboxylase may be formulated in a composition. In some embodiments, the pyridoxal-dependent decarboxylase is immobilized on a support as described herein in the context of the hydroxylating enzymes of the invention. In some other embodiments, the enzyme may be expressed *in situ* by a wild-type cell or a recombinant cell. It goes without saying that step (iii) is performed in conditions conducive for the pyroxydal-dependent decarboxylase activity, for instance in the presence of PLP and/or Dithiothreitol (DTT).

Furthermore, the method for preparing a hydroxylated diamino alkane according to the invention may comprise one or several additional steps. For instance, said methods may comprise one or several steps of purification which may be performed for instance between step (ii) and step (iii) or after step (iii). Additionally and/or alternatively, the process may comprise one or several additional reacting steps occurring before step (i) (e.g. so as to prepare the compound of formula (I)) and/or after step (iii).

The method for preparing a hydroxylated diamino alkane of the invention may be used for preparing a compound selected amoung (2S)-1,5-diamino-2-pentanol, 1,5-diamino-3-pentanol, (2S)-1,4-diamino-2-butanol, and a 1,5-diamino-pentanediol such as 1,5-diamino-pentane-2,3-diol and 1,5-diaminopentane-2,4-diol.

Accordingly, in a specific embodiment, the method of the invention is for preparing: 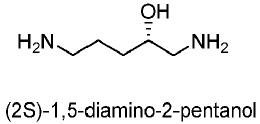 or a salt thereof,
said method comprising:
(i) preparing (3S)-hydroxy-L-lysine by contacting L-lysine with an enzyme capable of producing (3S)-hydroxy-L-lysine from L-lysine and comprising an amino acid sequence having at least 70% of sequence identity with SEQ ID NO:1,
(ii) decarboxylating (3S)-hydroxy-L-lysine formed in step (i) so as to obtain (2S)-1,5-diamino-2-pentanol, and
(iii) optionally, recovering and/or purifying the resulting (2S)-1,5-diamino-2-pentanol.

In an additional aspect, the method of the invention is for preparing: 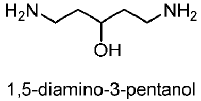 or a salt thereof, comprising:
(i) preparing (4R)-hydroxy-L-lysine by contacting L-lysine with an enzyme capable of producing (4R)-hydroxy-L-lysine from L-lysine and comprising an amino acid sequence having at least 70% of sequence identity with an amino acid sequence selected from SEQ ID NO:2, SEQ ID NO:3, SEQ NO:9 and SEQ NO:10,
(ii) decarboxylating (4R)-hydroxy-L-lysine formed in step (i) so as to obtain 1,5-diamino-3-pentanol,
(iii) optionally, recovering and/or purifying the resulting 1,5-diamino-3-pentanol.

In another aspect, the method of the invention is for preparing: 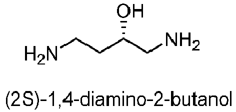 or a salt thereof, comprising:
(i) preparing (3S)-hydroxy-L-ornithine by contacting L-ornithine with an enzyme capable of producing (3S)-hydroxy-L-ornithine from L-ornithine and comprising an amino acid sequence having at least 70% of sequence identity with SEQ NO:4,
(ii) decarboxylating (S)-hydroxy-L-ornithine formed in step (i) so as to obtain (2S)-1,4-diamino-2-butanol, and
(iii) optionally, recovering and/or purifying the resulting (2S)-1,4-diamino-2-butanol.

In other aspects, the method of the invention is for preparing a 1,5-diamino pentanediol, said method comprising:
(i) preparing dihydroxy-L-lysine by contacting a hydroxy-L-lysine with an enzyme having a L-lysine hydroxylase activity and comprising an amino acid sequence having at least 70% of sequence identity with an amino acid sequence selecting from SEQ NO:1, SEQ ID NO:2, SEQ NO:3, SEQ NO:9 and SEQ NO:10, preferably SEQ ID NO:1, SEQ NO:2, and SEQ NO:3.
(ii) decarboxylating the dihydroxy-L-lysine formed in step (i) so as to obtain a 1,5-diamino pentanediol,
(iii) optionally, recovering and/or purifying the resulting 1,5-diamino pentanediol.

Examples of 1,5-diamino pentanediol which may be prepared by the method of the invention encompass, without being limited to:

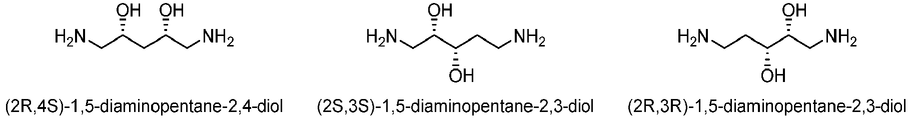

In a further aspect, the method of the invention is a method for preparing (2R,3R)-1,5-diamino-2,3-pentanediol or a salt thereof, comprising
(i) preparing (3S)-hydroxy-L-lysine by contacting L-lysine with an enzyme capable of producing (3S)-hydroxy-L-lysine from L-lysine and comprising an amino acid sequence having at least 70% of sequence identity with SEQ ID NO:1,
(ii) preparing (3R,4R)-dihydroxy-L-lysine by reacting (3S)-hydroxy-L-lysine obtained in step (i) with an enzyme capable of producing (4R)-hydroxy-L-lysine from L-lysine and comprising an amino acid sequence having at least 70% of sequence identity with an amino acid sequence selected from SEQ ID NO:2, SEQ ID NO:3, SEQ ID NO:9 and SEQ ID NO:10, preferably from SEQ ID NO:2, and SEQ ID NO:3,
(iv) decarboxylating (3R,4R)-dihydroxy-L-lysine formed in step (ii) so as to obtain (2R,3R)-1,5-diamino-2,3-pentanediol, and
(v) optionally, recovering and/or purifying the resulting (2R,3R)-1,5-diamino-2,3-pentanediol or salt thereof.

In some embodiments of the methods described hereabove, the step of decarboxylation is performed with a pyridoxal dependent decarboxylase, preferably comprising an amino acid sequence having a sequence identity of at least 70%, with the amino acid sequence of SEQ ID NO:11 or SEQ ID NO: 12.

### Further methods and uses of an enzyme of the invention

In an alternate aspect, it is also provided a method for hydroxylating L-arginine, comprising:
- contacting L-arginine with an enzyme having L-arginine hydroxylase activity and comprising an amino acid sequence having at least 70% of sequence identity with SEQ ID NO:4, and
- recovering and/or isolating hydroxylated L-arginine.

In a more specific aspect, said method is for preparing (3S)-hydroxy-L-arginine and the enzyme is capable for producing (3S)-hydroxy-L-arginine from L-arginine. Preferably, the enzyme of the invention has at least 80%, 85%, 90%, 93%, 95%, 96%, 97%, 98%, 99%, or 99.5% sequence identity with SEQ ID NO:4. The enzyme may be in a free state, immobilized on a support or expressed in situ by a cell or a host cell, as described herein.

It is further provided the use of an enzyme having L-arginine hydroxylase activity and comprising an amino acid sequence having at least 80% of sequence identity with SEQ ID NO:4, or a support on which said enzyme is immobilized, or a host cell or a wild-type cell capable for expressing said enzyme, for preparing hydroxylated L-arginine, preferably for preparing (3S)-hydroxy-L-arginine.

In a further aspect, it is also provided a method for oxidizing thia-L-lysine, comprising:
- contacting thia-L-lysine with an enzyme having L-lysine hydroxylase activity and comprising an amino acid sequence having at least 70% of sequence identity with SEQ ID NO:2 or SEQ ID NO:3, and
- recovering and/or isolating oxidized thia-L-lysine.

In a more specific aspect, said method is for preparing (3S)-hydroxy-L-arginine and the enzyme is capable for producing (3S)-hydroxy-L-arginine from L-arginine. Preferably, the enzyme of the invention has at least 80%, 85%, 90%, 93%, 95%, 96%, 97%, 98%, 99%, or 99.5% sequence identity with SEQ ID NO:4. The enzyme may be in a free state, immobilized on a support or expressed in situ by a cell or a host cell, as described herein

In an additional aspect, the invention also relates to a method for decarboxylating a hydroxylated L-lysine, including a dihydroxy-L-lysine, or a hydroxylated L-ornithine comprising reacting said hydroxylated L-lysine or L-ornithine with an enzyme comprising an amino acid sequence having at least 70% of sequence identity with SEQ ID NO:11 or SEQ ID NO:12 and capable of decarboxylating L-lysine or L-ornithine.

Further aspects and advantages of the present invention will be disclosed in the following experimental section, which should be regarded as illustrative and not limiting the scope of the present application.

**Table**

| **SEQ ID NO:** | |
|---|---|
| 1 | Amino acid sequence of ENZ1 from *Catenulispora acidiphila* |
| 2 | Amino acid sequence of ENZ2 from *Flavobacterium johnsoniae* |
| 3 | Amino acid sequence of ENZ3 from *Chitinophaga pinensis* |
| 4 | Amino acid sequence of ENZ4 from *Catenulispora acidiphila* |
| 5 | Nucleic acid sequence encoding SEQ ID NO: (genomic DNA) |
| 6 | Nucleic acid sequence encoding SEQ ID NO:2 (genomic DNA) |
| 7 | Nucleic acid sequence encoding SEQ ID NO:3 (genomic DNA) |
| 8 | Nucleic acid sequence encoding SEQ ID NO:4 (genomic DNA) |
| 9 | Amino acid sequence of ENZ5 from *Niastella koreensis* |
| 10 | Amino acid sequence of ENZ6 from *Flavobacterium sp.* |
| 11 | Amino acid sequence of pyridoxal-dependent decarboxylase (Decarboxy1) from *Flavobacterium johnsoniae* |
| 12 | Amino acid sequence of pyridoxal-dependent decarboxylase (Decarboxy2) from *Chitinophaga pinensis* |
| 13 | Nucleic acid sequence encoding SEQ ID NO:9 |
| 14 | Nucleic acid sequence encoding SEQ ID NO:10 |
| 15 | Nucleic acid sequence encoding SEQ ID NO:11 |
| 16 | Nucleic acid sequence encoding SEQ ID NO: 12 |

### EXAMPLES

### I. Cloning and enzyme production

All steps from primers purchase to biochemical assays were performed in 96 micro-wells. The choice of the primers was based on Primer3 program. Oligonucleotides were from Sigma-Genosys. Specific extensions are added to the primers for cloning into our plasmid pET22b(+) (Novagen) modified for ligation independent cloning. The forward primers introduced a hexahistidine sequence in the proteins after the initial methionine for purification purposes. Plasmid were then introduced into E. coli BL21(DE3) plysE strains (Invitrogen) for over-expression. Cell cultures, Isopropyl β-D-thiogalactopyranoside (IPTG) induction for protein production and cell extracts were then conducted (A. Kreimeyer et al. J. Biol. Chem. 2007, 10, 7191-7197). For the production of the enzymes used for screening substrate or hydroxylase activity, an adaption for 96-microwell plate conditions was needed. Briefly, a 1.6 mL cell culture was induced for recombinant protein production in each well. After centrifugation, cells were suspended in 300 µl of 50 mM HEPES buffer pH 7.5 containing 50 mM NaCl, 10% glycerol, 1 mM Pefabloc SC and 0.2 µL LysonaseTM bioprocessing reagent (Merck), and sonicated using an ultrasonic bath (Bronson 2510 Ultrasonic Cleaner). After centrifugation to clarify the cell extract, the supernatants were analyzed by sodium dodecyl sulfate-polyacrylamide gel electrophoresis (SDS-PAGE) to check for recombinant protein production. Protein concentrations were determined by the Bradford method with bovine serum albumin as the standard (Bio-Rad). The samples were stored at -80°C.

### II. Purification of enzymes

The enzymes were purified by passing the crude extract through a Ni-NTA column (QIAGEN), according to the manufacturer's instructions. Large scale purification of ENZ1, ENZ2, ENZ3, ENZ4 were done by nickel affinity purifications (A. Kreimeyer et al. J. Biol. Chem. 2007, 10, 7191-7197). Protein concentrations were determined by the Bradford method with bovine serum albumin as the standard (Bio-Rad). The samples were analyzed by sodium dodecyl sulfate-polyacrylamide gel electrophoresis (SDS-PAGE) using the Invitrogen NuPage. The purified proteins were stored at -80°C.

### III. Detection of hydroxylase activity

The enzymatic reactions were performed in 220 µL containing 1 mM α-ketoglutarate, 2.5 mM sodium ascorbate, 1 mM Mohr's salt, cell lysate from 0.04 mg/mL to 0.54 mg/mL protein, 5 mM substrate in 50 mM HEPES buffer pH 7.5 at room temperature for 18 h under orbital stirring. 90 µL of each well were transferred out to a 96 micro-well daughter plate. 130 µL of a mixture containing 600 µM NADH, 50 mM NH₄Cl, 18.18 µg/mL glutamate dehydrogenase in 50 mM TRIS buffer pH 7.5 were added. The oxidation of NADH, which absorbs at 340 nm, could be monitored to evaluate the remaining α-ketoglutarate by spectrophotometry.

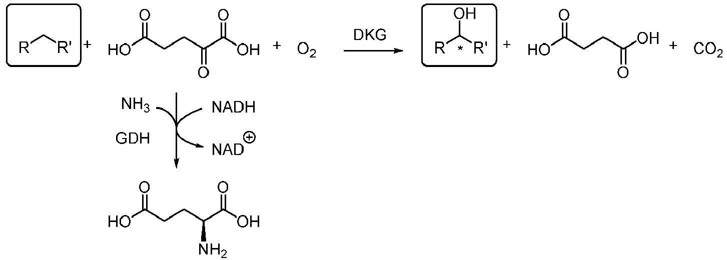

### IV. General protocols for the derivatization of amino acids and analysis of reaction media and/or derivatized amino acids by HPLC

### 1. Protocols for derivatization

### a. Protocol A: derivatization by Fmoc-Cl

Enzymatic reaction medium (0.2 µmole, leq.), borate buffer 0,2M pH 7.7 (80 µL) and Fmoc-Cl solution7.5 mM in acetone (100 µL, 0.75 µmole, 3.75 eq) were hand shaked in a closed 1.50 mL eppendorf for one minute. The aqueous layer was extracted with petroleum ether (3*1 mL) to eliminate excess of Fmoc-Cl. 70 µL of the aqueous phase were withdrawn, diluted with 70 µL of acetonitrile and injected into the HPLC. For substrates with two amine functions, 100 µL of Fmoc-Cl solution 15 mM were used.

*Conditions for HPLC analysis:* Atlantis® T3 (3 µm 4.5*150 mm) at 35°C with the gradient 50-86% eluent B (MeCN + 0.1% TFA) and eluent A (H₂O + 0.1% TFA) in 20 min at 1 mL/min.

*Retention times after derivatization:* L-lysine 15.06 min; 3-hydroxy-L-lysine 12.70 min; 4-hydroxy-L-lysine 12.66 min; 3,4 dihydroxy-L-lysine 11.26 min; L-ornithine 14.46 min; 3-hydroxy-L-ornithine 12.56 min.

### b. Protocol B: derivatization by 1-fluoro-2-4-dinitrophenyl-5-L-alanine amide (Marfey's reagent FDAA)

Enzymatic reaction medium (0.2 µmole, 1eq), 1% FDAA solution in acetone (50 µL, 1.5 µmoles, 7.5eq) and NaHCO₃ solution 1M (10 µl, 10 µmoles, 50 eq.) were stirred in a closed 1.50 mL eppendorf at 43°C, 1000 rpm for 90 minutes. HCl 1M (25 µL) was added. 100 µL were withdrawn, filtered and injected into the HPLC. For substrates with two amine functions, 120 µL of 1% FDAA in acetone were used.

*Conditions for HPLC analysis:* Atlantis® T3 (3 µm 4.5*150 mm) at 35°C the gradient 0% up to 50% in 17 and up to 70% in 2 minutes of eluent B (MeCN + 0.1% TFA) and eluent A (H₂O + 0.1 % TFA) at 1 mL/min.
*Retention times after derivatization:* L-arginine 12.70 min; 3-hydroxy-L-arginine 12.09 min.

### V. Selectivity of enzymes according to the invention

Enzymes (0.08 mg/mL) were screened against substrates (molar ratio α-ketoglutarate/substrate 15/10) in Hepes buffer 50 mM pH 7,5, with Mohr's salt at 1mM and without sodium ascorbate, at room temperature for three hours. The potential conversions of the substrates were determined by HPLC after Fmoc-Cl derivatization as section III here above.

| Substrates | | ENZ1 SEQ ID N°1 | ENZ2 SEQ ID NO:2 | ENZ3 SEQ ID NO:3 | ENZ4 SEQ ID NO:4 |
|---|---|---|---|---|---|
| L-lysine | 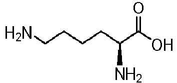 | **positive** | **positive** | **positive** | negative |
| L-ornithine | 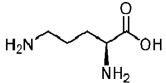 | negative | negative | negative | **positive** |
| L-arginine | 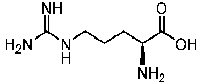 | negative | negative | negative | **positive** |
| (5R)- hydroxy-L-lysine | 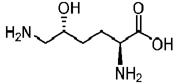 | **positive** | **positive** | **positive** | negative |
| (3S)- hydroxy-L-lysine | 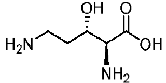 | - | **positive** | **positive** | negative |
| Thia-L-lysine | 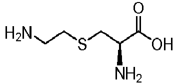 | negative | **positive** | **positive** | negative |
| D-lysine | 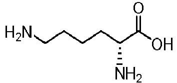 | negative | negative | negative | negative |
| D-ornithine | 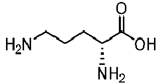 | negative | negative | negative | negative |
| D-arginine | 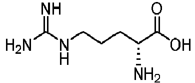 | negative | negative | negative | negative |
| L-citrulline | 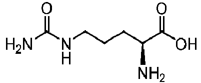 | negative | negative | negative | negative |
| 2-aminoheptanoic acid | 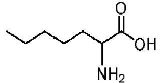 | negative | negative | negative | negative |
| 6-aminohexanoic acid | 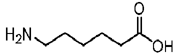 | negative | negative | negative | negative |
| 2-aminoadipic acid | 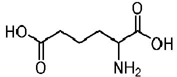 | negative | negative | negative | negative |
| Cadaverine | 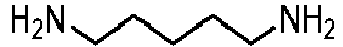 | negative | negative | negative | negative |
| Putrescine | 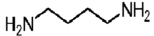 | negative | negative | negative | negative |
| L-(-)-α-Amino-ε-caprolactam | 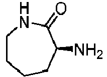 | negative | negative | negative | negative |
| L-Lysil-L-lysine | 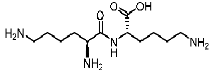 | negative | negative | negative | negative |
| Nε-Me-L-lysine | 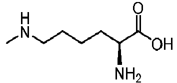 | negative | negative | negative | negative |
| Nε-Me3-L-lysine | 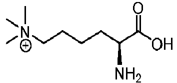 | negative | negative | negative | negative |
| L-lysine methyl ester | 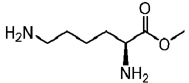 | negative | negative | negative | negative |
| L-ornithine methyl ester | 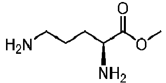 | negative | negative | negative | negative |
| L-arginine methyl ester | 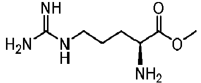 | negative | negative | negative | negative |
| Nα-acetyl-L-lysine | 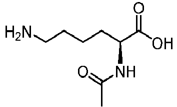 | negative | negative | negative | negative |
| Nα-acetyl-L-ornithine | 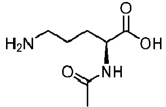 | negative | negative | negative | negative |
| Nα-acetyl-L-arginine | 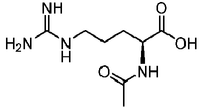 | negative | negative | negative | negative |
| β-L-lysine | 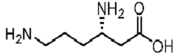 | negative | negative | negative | negative |

The hydroxylation capability of two other enzymes, namely ENZ6 (SEQ ID NO:10) and ENZ5 (SEQ ID NO:9) were also assessed. The amino sequence of ENZ6 has 79% of amino acid identity with SEQ ID NO:2 of ENZ2 while the amino sequence of ENZ5 has 72% of amino acid identity with SEQ ID NO:3 of ENZ3. The HPLC analysis of reaction media after derivatization demonstrated that both ENZ6 and ENZ5 were capable of hydroxylating L-lysine into hydroxy-L-lysine while the both enzymes are not able to convert L-ornithine. As shown in part VI, ENZ6 and ENZ5 provide (4R)-hydroxy-L-lysine from L-lysine similarly to ENZ2 and ENZ3. Thus ENZ6 and ENZ5 are likely to have the same substrate selectivity and provide similar products as ENZ2 and ENZ3.

### VI. Enzymatic hydroxylation of L-lysine, L-ornithine and L-arginine and characterization of the resulting hydroxylated products.

All reagents were purchased from commercial sources and used without additional purification. NMR spectra were recorded on a Bruker 300MHz spectrometer (Evry University, France). Chemical shifts (expressed in ppm) of ¹H and ¹³C NMR spectra were referenced to the solvent peaks δ H 7.24 and δ C 77.2 for CDCl₃, δ H 2.50 and δ C 39.5 for DMSO-d6, δ H 4.79 for D₂O. HRMS mass spectra were obtained with an electrospray source coupled with a time of flight analyser (LCT, Micromass). Thin-layer chromatography was performed with aluminium-backed sheets with silica gel 60 F₂₅₄ (Merck). Column chromatography was performed on a CombiFlash® Companion using Redwasep® *Rf* or GraceResolv™ Silica cartridges. HPLC analyses were performed on a Waters model 2795 liquid chromatograph provided with a Waters model 996 Photodiode Array Detector. HPLC preparative purifications were performed on a Shimadzu Model LC-20AP. *NB: The numbering of carbon atoms shown in the semi-structural chemical formula is for attributing NMR peaks and does not correlate with the conventional numbering of C-atoms used in the nomenclature.*

### 1. Isolation and characterization of the hydroxy-L-lysine formed by reaction of L-lysine with ENZ1 (SEQ ID NO:1)

The product resulting from the reaction of L-lysine with ENZ1 (SEQ ID NO:1) was (3S)-hydroxy-L-lysine.

The (3S)-hydroxy-L-lysine was characterized as its Boc derivative and the stereogenic C3 atom was determined by Mosher method.

### a. General protocol for hydroxylation with ENZ1 enzyme

Enzymatic reaction was carried out in 250 mL glass erlenmeyer flask for a total volume of 4*10 mL. Water (4.8 mL), Hepes buffer 1M pH 7.50 (0.5 mL, 50 mM), L-lysine 50 mM (2 mL, 10 mM), α-ketoglutarate 150 mM (1 mL, 15 mM), sodium ascorbate 100 mM (0.25 mL, 2.5 mM), ammonium Iron(II) sulfate hexahydrate 10 mM (1 mL, 1 mM) were poured into the erlenmeyer flask. The reaction was started by the enzyme addition (0.53 mg/mL, 0.45 mL, final concentration 0.024 mg/mL). The reaction mixture was orbitally shaken (300 rpm) at room temperature for 18h. The conversion of L-lysine into 3-hydroxy-L-lysine (94%) was determined by HPLC after derivatization with Fmoc-Cl according to protocol A.

The assembled reaction mixtures were acidified with 200 µL of TFA then centrifuged. The supernatant was collected and freeze-dried. The crude product was diluted in the minimum of water and eluted through a cation-exchange resin (DOWEX 50WX8-200) with a pH gradient from HCl 0.1 M to 3.5% NH₃ in water solution. The fractions containing the product were assembled and the solvent was evaporated under reduced pressure to give crude 3-hydroxy-L-lysine.

### b. General protocol for the purification of crude 3-hydroxy-L-lysine

### - Derivatization of 3-hydroxy-L-Lysine with tert-butoxycarbonyl group (Boc)

The crude residue from the enzymatic reaction (3-hydroxy-L-lysine/L-lysine 94/6, 0.376/0.024 mmole, 1 eq.) was solubilized in a 10% NaOH in water solution (400 µL, 0.8 mmole, 2 eq) and the solution was cooled to 0°C. Boc₂O 4M in ethanol (400 µL, 1.6 mmoles, 4 eq) was added dropwise and the reaction mixture was stirred at room temperature for 4 hours. 3mL of water were added and the aqueous layer was extracted with petroleum ether (3*3 mL) to eliminate the Boc₂O excess. The aqueous layer was acidified with HCl 1M until pH 2 and extracted with ethyl acetate (3*3 mL). The assembled organic layers were dried over anhydrous magnesium sulphate, filtered, and evaporated to give a yellowish oil. Purification by flash chromatography with the gradient 85-35% hexane + 0.3% HCO₂H/AcOEt + 0.3% HCO₂H in 20 minutes afforded *Nα*-Boc-*Nε*-Boc-(3S)-hydroxy-L-lysine as a yellowish oil (106 mg, 78%).
TLC Rf value 0.36 (petroleum ether /AcOEt 1/1+1% HCO₂H).
**¹H NMR** (CDCl₃, 300 MHz) δ (ppm): 1.36-1.44 (s, 18H, H11,H12), 1.49-1.72 (m, 4H, H4,H5), 3.02-3.14 (m, 2H, H6), 3.82-3.95 (m, 1H, H3), 4.26-4.36 (m, 1H, H2), 5.71-5.84 (m, 1H, C8N-H).
**¹³C NMR** (CDCl₃, 75 MHz) δ (ppm): 26.85 (C5), 28.49 (C11 or C12), 28.59 (C12 or C11), 30.19 (C4), 40.70 (C6), 58.61 (C2), 73.03 (C3), 80.61 (C9,C10), 156.47 (C7,C8), 173.22 (C1).

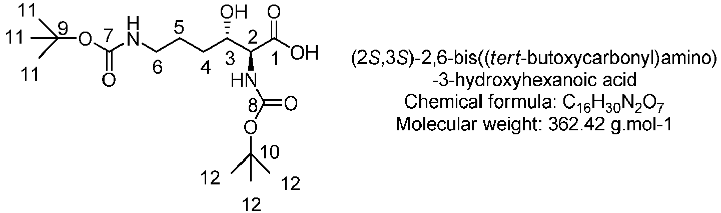

### - Cleavage of tert-butoxycarbonyl groups on ENZ1 product derivative

*Nα*-Boc-*Nε*-Boc-(3*S*)-hydroxy-L-lysine (42 mg, 0.116 mmole, 1eq.) was solubilized in HCl 4M in dioxane solution (345 µL, 1.38 mmole, 11.5 eq) and the reaction mixture was stirred at room temperature for one hour. The solvent was removed under reduced pressure and the residue was triturated in toluene (3*1 mL), diethyl ether (3*1 mL), dichloromethane (3*1 mL) successively and dried under vacuum overnight to afford the (3S)-hydroxy-L-lysine dihydrochloride as a white solid ( yield > 95%).

As mentioned above, the conformation of the stereogenic C3 atom was determined by Mosher method.
TLC Rf value: 0.31 AcOEt/acetone/H₂O/HCO₂H 5/3/2/2 (CTDD).
**¹H NMR** (D_{2O}, 300 MHz) δ (ppm): 1.56-1.92 (m, 4H, H4,H5), 2.95-3.05 (t, J= 7.2 Hz, 2H, H6), 3.98-4.02 (d, J= 3.3 Hz, 1H, H2), 4.04-4.11 (m, 1H, H3).**¹³C NMR** (D₂O with MeOH as standard, 75 MHz) δ (ppm): 24.20 (C5), 29.23 (C4), 39.61 (C6), 58.65 (C2), 69.55 (C3), 170.77 (C1).

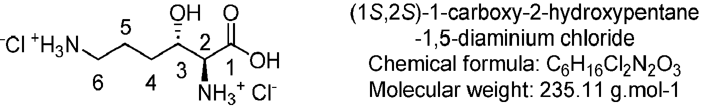

### 2. Isolation and characterization of the hydroxy-L-lysine formed by reaction of L-lysine with ENZ2 (SEQ ID NO:2) and ENZ3 (SEQ ID NO:3)

The product resulting from the reaction of L-lysine with ENZ2 (SEQ ID NO:2) and ENZ3 (SEQ ID NO:3) was in both case (4R)-hydroxy-L-lysine.

The (4R)-hydroxy-L-lysine was characterized as its cyclic derivative and the stereogenic C4 atom was determined by 2D ROESY NMR experiments.

### a. General protocol for L-lysine hydroxylation by ENZ2 (SEQ ID NO:2) or ENZ3 (SEQ ID NO:3)

The protocol used was the same as that described above for the hydroxylation by ENZ1 except that the enzyme concentrations used were different (ENZ2: 0.086 mg/mL; ENZ3: 0.031 mg/mL). Conversions were determined by HPLC after derivatization with Fmoc-Cl according to protocol A as described above. The conversion of L-lysine into (4R)-hydroxy-L-lysine was 91% with ENZ2 and 96% with ENZ1. Crude (4R)-hydroxy-L-lysine was obtained as a yellowish oil.

### b. General protocol for the purification of crude (4R)-hydroxy-L-lysine - derivatization with tert-butoxycarbonyl derivatization

The derivatization with Boc group was then performed as described above for (3S)-hydroxy-L-lysine with Boc₂O.

The crude yellowish oil was suspended in DCM overnight to ensure the total lactonization.

The resulting product was purified by flash chromatography with the gradient 85-35% hexane + 0.3% HCO₂H/AcOEt + 0.3% HCO₂H in 20 minutes, whereby di-tert-butyl (3S,5R)-5-(2-ammonioethyl)-2-oxotetrahydrofuran-3-amine was obtained as an uncolored oil.
ENZ2 product (92 mg, 71%); ENZ3 product (103 mg, 78%).
TLC Rf value 0.54 (petroleum ether /AcOEt 1/1+1% HCO₂H).
**¹H NMR** (CDCl₃, 300 MHz) δ (ppm): 1.38-1.47 (d, 18H, H11,H12), 1.74-2.05 (m, 3H, H3,H5), 2.76-2.90 (m, 1H, H3'), 3.23-3.35 (m, 2H, H6), 4.31-4.53 (br s, 2H, H2,H4), 4.65-4.82 (m, 1H, C7N-H), 5.00-5.14 (m,1H, C8N-H).
**¹³C NMR** (CDCl₃, 75 MHz) δ (ppm): 28.41 (C11 or C12), 28.53 (C12 or C11), 35.63 (C3 or C5), 36.40 (C5 or C3), 37.36 (C6), 51.41 (C2), 76.22 (C4), 79.66 (C9 or C10), 80.59 (C10 or C9), 155.51 (C7 or C8), 156.17 (C8 or C7), 174.91 (C1).

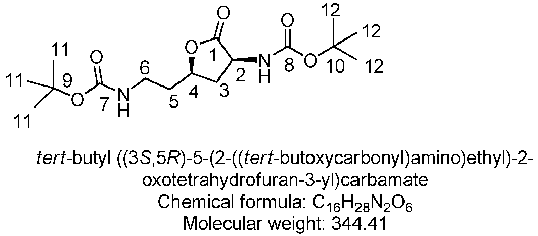

### - Cleavage of tert-butoxycarbonyl

Boc group was cleaved by HCl in dioxane using the standard protocol described here above for (3S)-hydroxy-L-lysine.

The (3S,5R)-5-(2-aminoethyl)-2-oxotetrahydrofuran-3-aminium chloride was obtained as white solid. Yield > 95%.
TLC Rf value: 0.35 (CTDD).
**N¹H MR** (DMSO, 300 MHz) δ (ppm): 1.91-2.16 (m, 3H, H5, H3), 2.63-2.77 (br s, 1H, H3'), 2.78-2.96 (m, 2H, H6), 4.33-4.48 (m, 1H, H2), 4.55-4.73 (m, 1H, H4).
**¹³C NMR** (DMSO, 75 MHz) δ (ppm): 32.00 (C3), 32.28 (C5), 35.15 (C6), 48.60 (C2), 75.69 (C4), 172.29 (C1).

The conformation of stereogenic C4 atom was determined by 2D ROESY NMR experiments.

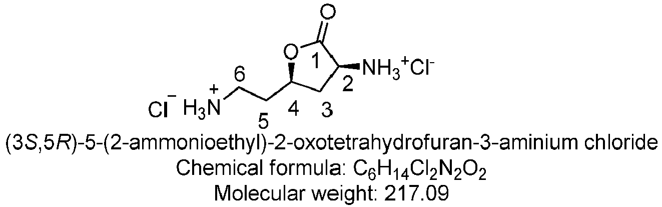

The corresponding (4R)-hydroxy-L-lysine can be obtained by hydrolysis of the lactone with a base (e.g. NaOH) or by treatment with silver acetate, (see Izumiya et al. Biochem 1965, 4, 11, 2501-2507).

### 3. Isolation and characterization of the hydroxy-L-lysine formed by reaction of L-lysine with ENZ5 (SEQ ID NO:9) and ENZ6 (SEQ ID NO:10)

As in the case of ENZ2 or ENZ3, the product resulting from the reaction of L-lysine with ENZ4 (SEQ ID NO:9) or ENZ5 (SEQ ID NO:10) was (4R)-hydroxy-L-lysine.

The protocol used was the same as that described for ENZ2 and ENZ3 except that cell lysate was used instead of purified enzymes.

### 4. Isolation and characterization of the hydroxy-L-ornithine formed by reaction of L-ornithine with ENZ4 (SEQ ID NO:4)

The product resulting from the reaction of L-ornithine with ENZ4 (SEQ ID NO:4) is (3S)-hydroxy-L-ornithine. The (3S)-hydroxy-L-ornithine was characterized as its DNB derivative. The stereogenic C3 atom was determined by comparison with published synthetic 3-hydroxy-L-ornithine (J. Org. Chem. 1987, 52, 2179).

### a. Protocol of L-ornithine hydroxylation by ENZ4 (SEQ ID NO:4)

Enzymatic reaction was carried out in one 250 mL glass erlenmeyer flask for a total volume of 10 mL for FDNB derivatization or 4*10 mL for product isolation.

The protocol for L-ornithine hydroxylation was the same as that described above for the hydroxylation of L-lysine by ENZ1 except that the enzyme concentration was 0.595 mg/mL for ENZ4 and the reaction was carried without sodium ascorbate. The conversion of L-ornithine into hydroxylated product (68%) was determined by HPLC after derivatization with Fmoc-Cl according to protocol A.

The hydroxylated product was obtained as a crude yellowish oil.

### b. Determination of the stereochemistry

The hydroxylated product was characterized as its DNB derivative by comparison with (3S)-hydroxy-L-ornithine DNB derivative NMR data.

For FDNB derivatization, a fraction of the reaction medium was acidified with 125 µL of 1% TFA in water solution, centrifugated, the supernatant was collected and freeze-dried.

The resulting residue (3-hydroxy-L-ornithine/L-ornithine 5.5/4.5 0.055/0.045 mmole,1eq. was solubilized in a 2% NaHCO₃ solution in water (10 mL, 2.4moles, 24 eq.) and a 0.25% FDNB solution in ethanol (60 mL, 0.7 mmoles, 7eq) was added. The reaction mixture was stirred at 50°C for 2 hours and then acidified with HCl 1M (1mL). The solvents were removed under reduced pressure to give the crude product as a yellow powder. Purification by preparative HPLC using Xbridge prep C18 5µm OBD 19*150 mm with gradient 0-65% Eluent B (MeCN+ 0.1% TFA) and eluent A (H₂O +0.1% TFA) in 17 minutes afforded (2S,3R)-2,5-bis((2,4-dinitrophenyl)amino)-3-hydroxypentanoic acid as a yellow solid (18.7 mg, 71%, rt: 12.76 min).

**NMR ¹H** (DMSO d6, 300 MHz) δ (ppm): 1.85-2.09 (m, 2H, H4), 3.55-3.72 (m, 2H, H5), 4.06-4.15 (m, 1H, H3), 4.68-4.78 (dd, J= 7.6 Hz, J= 2.7 Hz, 1H, H2), 7.18-7.33 (m, 2H, H7,H13), 8.20-8.32 (m, 2H,H8,H14), 8.82-8.99 (dd, J= 2.7 Hz, J= 10.4 Hz, 2H, H10, H16), 9.00-9.08 (t, J=5.8 Hz, 1H, C5N-H), 9.14-9.21 (d, J= 7.6 Hz, 1H, C2N-H).

**NMR ¹³C** (DMSO, 75 MHz) δ (ppm): 31.22 (C4), 40.53 (C5), 60.46 (C3), 69.27 (C2), 115.12 (C7 or C13), 116.25 (C13 or C7), 123.43 (C10 or C16), 123.57 (C16 or C10), 129.74 (C8 or C14), 129.77 (C14 or C8), 129.91 (C9 or C15), 130.23 (C15 or C9), 134.65 (C11 or C17), 135.31 (C17 or C11), 147.28 (C6 or C12), 148.02 (C12 or C6), 170.06 (C1).

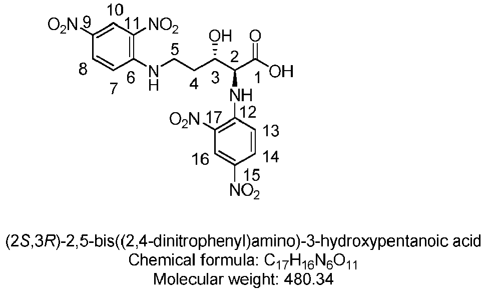

### c. General protocol for the purification of crude (3S)-hydroxy-L-ornithine

The four reaction media (4*10 mL) were assembled, acidified with 200 µL of TFA, centrifuged and the supernatant was collected and freeze-dried.

The resulting residue was purified according to the general protocol described above for the crude (3S)-hydroxy-L-lysine. Briefly, the residue was derivatized with a Boc group. The resulting product was purified by flash chromatography with the gradient 85-35% hexane + 0.3% HCO₂H/AcOEt + 0.3% HCO₂H in 20 minutes afforded *Nα*-Boc-*Nδ*-Boc-(3S)-hydroxy-L-ornithine as a yellowish oil (58 mg, 62%).
TLC Rf value 0. 25 (petroleum ether /AcOEt 1/1+1% HCO₂H).
**¹H NMR** (CDCl₃, 300 MHz) δ (ppm): 1.32-1.46 (s, 18H, H10, H11), 1.64-1.81 (m, 2H, H4), 3.10-3.38 (m, 2H, H5), 3.89-4.01 (m, 1H, H3), 4.25-4.35 (m, 1H, H2), 5.71-5.84 (m, 1H, N-H).
**N¹³C MR** (CDCl₃, 75 MHz) δ (ppm): 28.47 (C10 or C11), 28.56 (C11 or C10), 33.46 (C4), 37.69 (C5), 58.46 (C2), 70.91 (C3), 80.66 (C8, C9), 156.43 (C6, C7), 172.93 (C1).

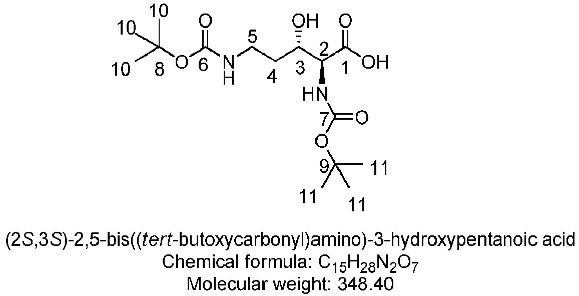

The cleavage of Boc group was performed as described above for hydroxy-L-lysine, by reacting *Nα*-Boc-N*δ-*Boc-(3S)-hydroxy-L-omithine with HCl 4M in dioxane solution.The (3S)-3-hydroxy-L-ornithine was obtained as a white solid (yield > 95%).
TLC Rf value: 0.31 (CTDD).
**¹H NMR** (D₂O, 300 MHz) δ (ppm): 1.86-2.12 (m, 2H, H4), 3.08-3.21 (m, 2H, H5), 4.09-4.14 (d, J= 3.3 Hz, 1H, H2), 4.18-4.27 (m, 1H, H3).
**¹³C NMR** (D₂O with MeOH as standard, 75 MHz) δ (ppm): 30.03 (C4), 37.65 (C5), 39.74 (C6), 58.32 (C2), 68.05 (C3), 170.04 (C1).

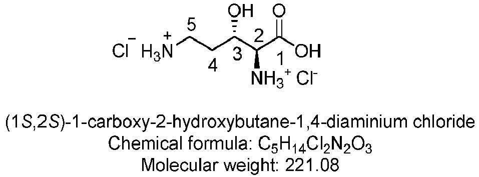

### 5. Isolation and characterization of the hydroxy-L-arginine formed by reaction of L-ornithine and L-arginine with ENZ4 (SEQ ID NO:4)

The protocol for L-arginine hydroxylation was the same as that used for the hydroxylation of L-lysine by ENZ1 except that the enzyme concentration was 0.595 mg/mL for ENZ4 and the reaction was carried without sodium ascorbate. The conversion of L-arginine into hydroxylated product (50%) was determined by HPLC after after derivatization with FDAA according to protocol B.

The hydroxylated product was characterized as its DNB derivative, as described above for hydroxylated L-ornithine (conditions : 0.25% FDNB solution in ethanol (30 mL, 0.35 mmole, 3.5eq).

The resulting product was purified by preparative HPLC using Xbridge prep C18 5 µm OBD 19*150 mm with 0-100% in 20 minutes of eluent B (MeCN) and eluent A (H₂O) afforded (2S,3S)-2-((2,4-dinitrophenyl)amino)-5-guanidino-3-hydroxypentanoic acid as a yellow solid (13.3 mg, 75%, rt: 11.00 min).
**¹H NMR** (DMSO d6, 300 MHz) δ (ppm): 1.58-1.77 (m, 2H, H4), 3.04-3.28 (m, 2H, H5), 4.08-4.18 (br s, 1H, H3), 4.27-4.35 (dd, J= 7.2 Hz, J= 3.1 Hz, 1H, H2), 5.20-5.28 (br s, 1H, OH), 7.32-7.39 (d, J= 9.9 Hz, 1H, H8), 7.46-7.67 (br s, 4H, H₂N-C=NH₂⁺), 8,12-8,20 (dd, J= 9.9 Hz, J2= 2.7 Hz, 1H, H9), 8.71-8.80 (m, 1H, C6-NH), 8.85-8.89 (d, J= 2.7 Hz, 1H, H11), 9.53-9.61 (d, J= 7.2 Hz, 1H, C2-NH).
**¹³C NMR** (DMSO, 75 MHz) δ (ppm): 31.14 (C4), 37.83 (C5), 62.68 (C2), 69.76 (C3), 117.20 (C8), 123.55 (C11), 129.13 (C9), 129.46 (C10), 134.29 (C12), 147.98 (C7), 157.18 (C6), 171.04 (C1).

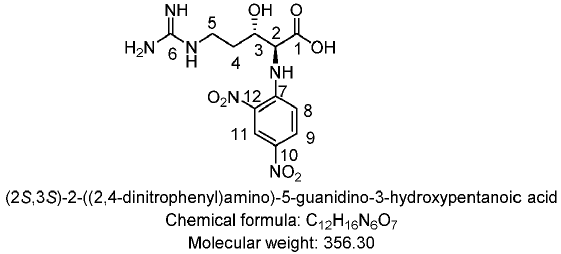

### 6. Isolation and characterization of the dihydroxy-L-lysine formed by cascade reaction of L-lysine with ENZ1 (SEQ ID NO:1) and ENZ2 (SEQ ID NO:2)

The product resulting from the cascade reaction of L-lysine with ENZ1 (SEQ ID NO:1) and ENZ2 (SEQ ID NO:2) was (3R,4R)-3,4-dihydroxy-L-lysine.

### a. L-lysine dihydroxylation

Enzymatic reaction was carried out in 250 mL glass erlenmeyer flask for a total volume of 4*10 mL. Water (5.6 mL), Hepes buffer 1M pH 7.50 (0.5 mL, 50 mM), L-lysine 10 mM (1 mL, 10 mM), α-ketoglutarate 150 mM (1 mL, 15 mM), sodium ascorbate 100 mM (0.25 mL, 2,5 mM), ammonium Iron(II) sulfate hexahydrate 10 mM (1 mL, 1 mM) were poured into the erlenmeyer. The reaction was started by ENZ1 addition (1.99 mg/mL, 0.65 mL, final concentration 0.13 mg/mL). The reaction mixture was orbitally shaken (600 rpm) at room temperature for 3h then α-ketoglutarate 150 mM (1.37 mL), sodium ascorbate 100 mM (0.34 mL), ammonium Iron(II) sulfate hexahydrate 100 mM (0.137 mL) were added followed by ENZ2 addition (2.29 mg/mL, 1.85 mL). The reaction mixture was orbitally shaken (600 rpm) at room temperature for 18 h. The conversion (90%) was determined by HPLC after derivatization with Fmoc-Cl according to protocol A. The assembled reaction mixtures were acidified with 200 µL of TFA then centrifuged. The supernatant was collected and freeze-dried to give crude 3,4-di-hydroxy-L-lysine.

### b. tert-butoxycarbonyl derivatization

The derivatization with Boc group was then performed as described above for (3S)-hydroxy-L-lysine The crude yellowish oil was suspended in DCM overnight to ensure the total lactonization. The purification was performed by flash chromatography with the gradient 75-25% hexane + 0.3% HCO₂H/AcOEt + 0.3% HCO₂H in 20 minutes afforded tert-butyl ((3S,4R,SR)-5-(2-((tert-butoxycarbonyl)amino)ethyl)-4-hydroxy-2-oxotetrahydrofuran-3-yl)carbamate (76 mg, 59%) as an uncolored oil.
TLC Rf value 0.54 (petroleum ether /AcOEt 1/1+1% HCO₂H)
**¹H NMR** (CDCl₃, 300 MHz) δ (ppm): 1.37-1.48 (d, 18H, H11,H12), 1.92-2.18 (m, 2H, H5), 3.15-3.36 (m, 2H, H6), 3.51-4.03 (br s, 1H, OH), 4.37-4.70 (m, 3H, H2,H3,H4), 4.91-5.16 (m, 1H, C7-NH), 5.30-5.49 (m,1H, C8-NH).
**¹³C NMR** (CDCl₃, 75 MHz) δ (ppm): 28.45 (C11 or C12), 28.57 (C12 or C11), 28.67 (C5), 36.77 (C6), 55.82 (C3), 69.91 (C2), 80.00 (C4), 80.26 (C9 or C10), 81.15 (C10 or C9), 156.00 (C7 or C8), 156.79 (C8 or C7), 174.11 (C1).

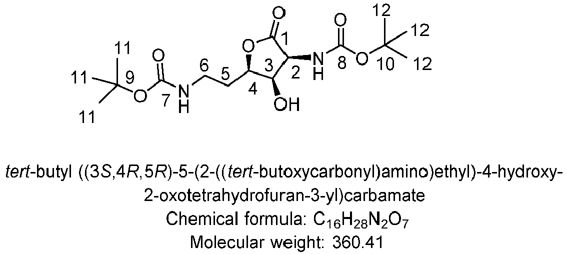

### c. tert-butoxycarbonyl cleavage

The (3S,4R,5R)-5-(2-ammonioethyl)-4-hydroxy-2-oxotetrahydrofuran-3-aminium chloride was obtained as white solid ( yield > 95) after the cleavage of Boc groups by HCl 4M in dioxane.
TLC Rf value: 0.35 (CTDD)
**¹H NMR** (DMSO, 300 MHz) δ (ppm): 1.91-2.13 (m, 2H, H5), 2.81-3.07 (m, 2H, H6), 4.46-4.58 (m, 2H, H2, H3), 4.60-4.69 (m, 1H, H4), 6.59-6.81 (br s, 1H, OH), 8.06-8.38 (br s, 3H, C6-NH₃+), 8.46-9.06 (br s, 3H, C2-NH₃+).
**¹³C NMR** (DMSO, 75 MHz) δ (ppm): 26.35 (C5), 35.31 (C6), 52.55 (C3), 67.70 (C2), 80.19 (C4), 171.87 (C1).

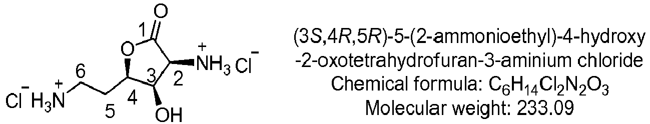

The corresponding (3R,4R)-dihydroxy-L-lysine can be obtained by hydrolysis of the lactone with a base (e.g. NaOH) or by treatment with silver acetate, (see Izumiya et al. Biochem 1965, 4, 11, 2501-2507).

### VII. Enzymatic synthesis of (2S)-1,5-diamino-2-pentanol, 1,5-diamino-3-pentanol, (S)-1,4-diamino-2-butanol and (2R,3R)-1,5-diamino-2,3-pentanediol.

The enzymatic synthesis comprised a step of hydroxylation of L-lysine or **L-ornithine** by an enzyme according to the invention and then the cleavage of the carboxyl group by using an appropriate decarboxylase.

The conversions were determined by HPLC after Fmoc-Cl derivatization (protocol A). Atlantis T3 (3µm 4.5*150 mm) at 35°C with the gradient 50-86% eluent B (MeCN + 0.1% TFA) and eluent A (H₂O + 0.1% TFA) in 20 min at 1 mL/min at 30°C.

### 1. (2S)-1,5-diamino-2-pentanol.

L-lysine was converted into (*3S*)-3-hydroxy-L-lysine by ENZ1 in conditions described in part V.

30 µL pyridoxal 5'-phosphate (PLP) 10 mM, 3 µL dithiothreitol (DTT) 100 mM and Decarboxyl (or Decarboxy2) 50 µL 1.5 mg/mL were then added in the reaction medium. The mixture was stirred (1000 rpm) for 18h at 37 °C. Retention time of (2S)-1,5-diamino-2-pentanol after derivatization with Fmoc: 14.82 min.

### Conversion >95%.

Figure 1 shows the monitoring of the enzymatic conversion of L-lysine into (2S)-1,5-diamino-2-pentanol by HPLC according to protocol A (Fmoc derivatization). From bottom to top: HPLC chromatogram obtained for the reaction medium before hydroxylation of L-lysine with ENZ1, HPLC chromatogram of the reaction medium after hydroxylation and HPLC chromatogram obtained for the reaction medium after decarboxylation.

### 2. 1,5-diamino-3-pentanol.

Lysine was converted into (4R)-4-hydroxy-L-lysine in conditions described in part V 30 µL PLP 10 mM, 3 µL DTT 100 mM and Decarboxyl(or Decarboxy2) 50 µL 1.5 mg/mL were then added in the reaction medium to perform decarboxylation. The mixture was stirred (1000 rpm) for 18h at 37 °C. Retention time of 1,5-diamino-3-pentanol after derivatization with Fmoc: 15.12 min. Conversion 100%.

Figure 2 shows the monitoring of the enzymatic conversion of L-lysine into 1,5-diamino-3-pentanol by HPLC according to protocol A (Fmoc derivatization). From bottom to top: chromatogram obtained for the reaction medium before hydroxylation (starting product), chromatogram of the reaction medium after hydroxylation of L-lysine with ENZ2 and chromatogram obtained for the reaction medium after decarboxylation (final product). The peak at 13.78 min (middle chromatogram) corresponds to a Fmoc-derivativized impurity present in purified Enz2 as evidenced by the chromatogram of the purification medium (data not shown).

### 3. (2S)-1,4-diamino-2-butanol

Ornithine was converted into (3S)-3-hydroxy-L-ornithine in conditions described in part V. 30 µL PLP 10 mM, 3 µL DTT 100 mM and Decarboxyl (or Decarboxy2) 50 µL (lysat) were then added to perform decarboxylation. The mixture was stirred (1000 rpm) for 18h at 37 °C. Retention time of (2S)-1,4-diamino-2-butanol after derivatization with Fmoc: 14.68 min. Conversion 50%.

Figure 3 shows the monitoring of the enzymatic conversion of L-ornithine into (2S)-1,4-diamino-2-butanol by HPLC according to protocol A (Fmoc derivatization). From bottom to top: chromatogram obtained for the reaction medium before hydroxylation (starting product), chromatogram of the reaction medium after hydroxylation of L-ornithine with ENZ4 and chromatogram obtained for the reaction medium after decarboxylation (final product). The extra peaks correspond to Fmoc-derivatized impurities from cell lysates (data not shown).

### 4. (2R,3R)-1,5-diamino-2,3-pentanediol.

Lysine was converted into (3R,4R)-3,4-dihydroxy-L-lysine in conditions described in part V, by cascade reaction with ENZ1 (SEQ NO:1) and ENZ2 (SEQ NO:2).

The volume of the reaction medium was reduced to 180 µL using a speedvac.
26 µL PLP 10 mM, 2.6 µL DTT 100 mM and Decarboxyl (or Decarboxy2) 50 µL (lysat) were then added to perform decarboxylation. The mixture was stirred (1000 rpm) for 18h at 37 °C. Retention time of (2R,3R)-1,5-diamino-2,3-pentanediol after derivatization with Fmoc: 12.62 min. Conversion >90%.

Figure 4 shows the monitoring of the enzymatic conversion of L-lysine into (2R,3R)-1,5-diamino-2,3-pentanediol by HPLC according to protocol A (Fmoc derivatization). From bottom to top: chromatogram obtained for the starting reaction medium, chromatogram obtained after hydroxylation with ENZ4 , chromatogram obtained for the reaction medium after the 2^{nd} hydroxylation and chromatogram obtained for the reaction medium after decarboxylation (final product). The peak at 12.57 min (top chromatogram) corresponds to a contaminant present within the cell lysate of the decarboxylase.

## Claims

1. A method for hydroxylating a compound of formula (I), or a salt thereof: 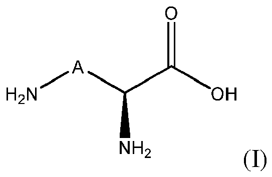 wherein A is -(CH₂)ₙ-X-(CH₂)ₚ- with X is CH₂, or CH(OH), n is an integer from 0 to 4, preferably from 1 to 4, and p is an integer from 0 to 4 with proviso that 0≤ n+p ≤ 4 wherein said method comprises
- contacting the compound of formula (I) or a salt thereof with an enzyme having a L-lysine or L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO: 10, SEQ ID NO:1, and SEQ ID NO:4; and
- optionally recovering the hydroxylated compound.

2. The method of claim 1, wherein:
- the compound of formula (I) is such that n is an integer from 1 to 3 and p is an integer from 0 to 2 with proviso that n + p =3,
- the enzyme has a L-lysine hydroxylase activity and comprises an amino acid sequence having at least 70% of sequence identity with an amino acid sequence selected from the group consisting of SEQ ID NO:2, and SEQ ID NO:3, SEQ ID NO:9, SEQ ID NO:10, and SEQ ID NO:1.
- the hydroxyl group is introduced on a carbon position of moiety A of the compound of formula (I)

3. The method of claim 1, which is **characterized by** one of the following combination of features:
- Said method is for preparing (4R)-hydroxy-L-lysine and comprises contacting L-lysine with an enzyme capable of producing (4R)-hydroxy-L-lysine from L-lysine and comprising an amino acid sequence having at least 70% of sequence identity with SEQ ID NO:2, SEQ ID NO:3, SEQ ID NO:9 or SEQ ID NO:10; or
- Said method is for preparing (3S)-hydroxy-L-lysine and comprises contacting L-lysine with an enzyme capable of producing (3S)-hydroxy-L-lysine from L-lysine and comprising an amino acid sequence having at least 70% of sequence identity with SEQ ID NO:1, or
- Said method is for preparing (3S)-hydroxy-L-ornithine and comprises contacting L-ornithine with an enzyme capable of producing (3S)-hydroxy-L-ornithine from L-ornithine and comprising an amino acid sequence having at least 80% of sequence identity with SEQ ID NO:4.

4. The method of any one of claims 1 to 3 wherein said enzyme comprises an amino acid sequence having at least 90%, preferably at least 95%, more preferably at least 99% of sequence identity with an amino acid sequence selected from the group consisting of SEQ ID NO:2, SEQ ID NO:3, SEQ ID NO:9, SEQ ID NO: 10, SEQ ID NO:1, and SEQ ID NO:4.

5. The method of any one of claims 1-4 wherein said enzyme and said compound of formula (I) are contacted in the presence of a source of Fe²⁺ and in the presence of an α-keto acid or a salt thereof.

6. A recombinant nucleic acid construct or vector comprising
- an nucleic acid sequence encoding an enzyme having a L-lysine or a L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO: 10, SEQ ID NO:1, and SEQ ID NO:4, and
- one or more control sequences which is/are operably linked to said nucleic acid sequence and that is/are capable to direct or control the production of the enzyme in a host cell

7. A recombinant host cell comprising the nucleic acid construct or vector as defined in claim 6.

8. A method for producing an enzyme having a L-lysine or a L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO:10, SEQ ID NO:1, and SEQ ID NO:4, said method comprising:
- cultivating a cell, which in its wild-type form is capable of producing the said enzyme, or a recombinant host cell of claim 7, in conditions conducive for production of the said enzyme, and
- recovering and/or purifying the said enzyme.

9. A solid support on which is immobilized an enzyme having a L-lysine or a L-ornithine hydroxylase activity and comprising an amino acid sequence having at least 70% of sequence identity with an amino acid sequence selected from the group consisting of SEQ ID NO:1, SEQ ID NO:2, SEQ ID NO:3, SEQ ID NO:9, SEQ ID NO:10, and SEQ ID NO:4.

10. A composition comprising an isolated enzyme having a L-lysine or a L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO:10, SEQ ID NO:1 and SEQ ID NO:4, and an excipient.

11. A kit for hydroxylating a compound of formula (I) as defined in claim 1 comprising: an isolated or recombinant enzyme having a L-lysine or L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO: 10, SEQ ID NO:1 and SEQ ID NO:4, a solid support of claim 9, a composition of claim 10, or a recombinant host cell of claim 7.

12. Use of an enzyme having a L-lysine or a L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO: 10, SEQ ID NO:1 and SEQ ID NO:4, a solid support of claim 9, a kit of claim 11, a composition composition of claim 10 or a recombinant host cell of claim 7 for hydroxylating a compound of formula (I) : 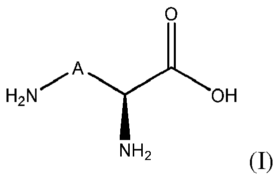 wherein A is -(CH₂)ₙ-X-(CH₂)ₚ- X is CH₂, or CH(OH), n is an integer from 0 to 4, preferably from 1 to 4, and p is an integer from 0 to 4 with proviso that 0≤ n+p ≤ 4, or a salt thereof.

13. A method for producing a compound of interest comprising:
- preparing a hydroxylated compound from a compound of formula (I) as defined in claim 1 by the method as defined in any one of claims 1-5, and
- producing the compound of interest from said hydroxylated compound.

14. A method for preparing a hydroxylated diamino alkane, said method comprising:
(i) providing a compound of formula (I): 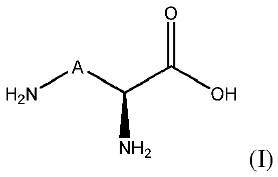 wherein A is -(CH₂)ₙ-X-(CH₂)ₚ- with X is CH₂, or CH(OH) and n is an integer from 0 to 4, preferably from 1 to 4, and p is an integer from 0 to 4 with proviso that 0≤ n+p ≤ 4, preferably n+p = 2 or 3,
(ii) hydroxylating compound of formula (I) by contacting said compound with an enzyme having a L-lysine or L-ornithine hydroxylase activity and comprising an amino acid sequence having at least 70% of sequence identity with an amino acid sequence selected from the group consisting of SEQ ID NO:2, SEQ ID NO:3, SEQ ID NO:9, SEQ ID NO: 10, SEQ ID NO:1 and SEQ ID NO:4,
(iii) decarboxylating the compound obtained in step (ii), preferably by using a decarboxylase, and
(iv) optionally recovering the resulting hydroxylated diamino alkane.

15. The method of claim 14, wherein the hydroxylated diamino alkane is selected from the group consisting (2S)-1,5-diamino-2-pentanol, 1,5-diamino-3-pentanol, (2S)-1,4-diamino-2-butanol, and a 1,5 diamino pentanediol and salts thereof.
